(19) Europäisches Patentamt
European Patent Office
Office européen des brevets

(11) **EP 3 068 879 B1**

(12) **EUROPEAN PATENT SPECIFICATION**

(45) Date of publication and mention
of the grant of the patent:
**25.12.2019 Bulletin 2019/52**

(51) Int Cl.:
*C12N 9/96* [(2006.01)]      *C12N 9/98* [(2006.01)]
*C11D 3/386* [(2006.01)]      *C07K 1/00* [(2006.01)]

(21) Application number: **14812339.1**

(22) Date of filing: **14.11.2014**

(86) International application number:
**PCT/US2014/065612**

(87) International publication number:
**WO 2015/073772 (21.05.2015 Gazette 2015/20)**

(54) **STABLE ENZYMES BY GLYCATION REDUCTION**

STABILE ENZYME DURCH GLYKIERUNGSREDUKTION

ENZYMES STABLES OBTENUES PAR RÉDUCTION DE GLYCATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2013 US 201361904378 P
19.12.2013 US 201361918396 P**

(43) Date of publication of application:
**21.09.2016 Bulletin 2016/38**

(73) Proprietor: **Danisco US Inc.
Palo Alto, California 94304 (US)**

(72) Inventors:
• **GEBERT, Mark S.**
**Palo Alto, CA 94304 (US)**
• **DALSGAARD, Soren**
**Palo Alto, CA 94304 (US)**
• **ORTIZ-JOHNSON, Mariliz**
**Palo Alto, CA 94304 (US)**
• **GARSKE, Adam L.**
**Palo Alto, CA 94304 (US)**
• **DALE, Douglas A.**
**Palo Alto, CA 94304 (US)**

(74) Representative: **Mewburn Ellis LLP
City Tower
40 Basinghall Street
London EC2V 5DE (GB)**

(56) References cited:

EP-A1- 0 231 729       EP-A1- 1 996 028
WO-A1-97/39116        WO-A1-2011/117397
WO-A2-98/54980        WO-A2-2007/044968
WO-A2-2007/113292      WO-A2-2009/129489
WO-A2-2013/119468      GB-A- 2 167 758
US-A- 4 371 557        US-A1- 2010 083 392

• **PHILIPPE RONDEAU ET AL: "The glycation of albumin: Structural and functional impacts", BIOCHIMIE, MASSON, PARIS, FR, vol. 93, no. 4, 7 December 2010 (2010-12-07), pages 645-658, XP028172251, ISSN: 0300-9084, DOI: 10.1016/J.BIOCHI.2010.12.003 [retrieved on 2010-12-16]**
• **SAJILATA M G ET AL: "Resistant Starch - A Review", COMPREHENSIVE REVIEWS IN FOOD SCIENCE AND FOOD SAFETY, INSTITUTE OF FOOD TECHNOLOGISTS, CHICAGO, IL, US, vol. 5, 1 January 2006 (2006-01-01), pages 1-17, XP002504477, ISSN: 1541-4337, DOI: 10.1111/J.1541-4337.2006.TB00076.X**
• **ANNETTE T. LEE ET AL: "Role of Glycation in Aging", ANNALS OF THE NEW YORK ACADEMY OF SCIENCES, vol. 663, no. 1 Aging and Cel, 1 November 1992 (1992-11-01), pages 63-70, XP055169448, ISSN: 0077-8923, DOI: 10.1111/j.1749-6632.1992.tb38649.x**
• **SINGH R ET AL: "Advanced glycation end-products: A review", DIABETOLOGIA, SPRINGER, BERLIN, DE, vol. 44, no. 2, 1 February 2001 (2001-02-01), pages 129-146, XP002228061, ISSN: 0012-186X, DOI: 10.1007/S001250051591**

**Description**

**FIELD OF THE INVENTION**

This disclosure is directed towards improved enzymes, and enzyme formulations.

**BACKGROUND OF THE INVENTION**

The use of active agents, such as enzymes, in detergents, foods, and animal feed has become a common practice. There is an ongoing need in these industries for stable enzyme granules that maintain activity after being subjected to harsh conditions.

Approaches to avoid the problem of irreversibly inactivating enzymes or reducing the activity of the enzyme in industrial processes include identifying new sources of an enzyme (e.g. the identification of a known enzyme in an extreme thermophile microorganism) or identifying means to stabilize known enzymes. Klibanov, 1983, (Stabilization of Enzymes against Thermal Inactivation, Advances in Applied Microbiology, volume 29, page 1-28) discloses that there are three basic means for stabilizing enzymes: (1) immobilization, (2) chemical modification and (3) inclusion of additives. While previous formulation approaches have made some progress in this area (see for example WO9854980, WO9739116, WO2007044968), and EP1996028) the present teachings make an additional advance in overcoming some of these problems by recognizing that for certain enzymes reduced glycation can improve enzyme stability.

Phytase variants are known in the art, see, for example, WO2011/117397, US2010/083392A1 and WO2009/129489A2. Enzyme granules and compositions are also known, see for example, GB2167758A, WO2013/119468 and WO2007/113292A2.

EP0231729A1 and US4371557A relate to the reduction of Mailiard reactions in food products. Rondeau et al., Biochimie, The glycation of albumin: Structural and functional impacts (2010), discusses glycation of albumin.

**SUMMARY OF THE INVENTION**

The present teachings provide improved enzymes, formulated enzymes, and methods of making them.

**BRIEF DESCRIPTION OF THE FIGURES**

**Figures 1A-B** depict illustrative data according to the present teachings.
**Figures 2A-D** depict illustrative data according to the present teachings.
**Figure 3** depicts illustrative data according to the present teachings.
**Figure 4** depicts illustrative data according to the present teachings.
**Figure 5** depicts illustrative data according to the present teachings.
**Figure 6** depicts illustrative data according to the present teachings.
**Figures 7A-B** depict illustrative data according to the present teachings.
**Figure 8** depicts illustrative data according to the present teachings.
**Figure 9** depicts illustrative data according to the present teachings.
**Figure 10** depicts illustrative data according to the present teachings.
**Figure 11** depicts illustrative data according to the present teachings.
**Figure 12** depicts illustrative data according to the present teachings.
**Figure 13** depicts illustrative data according to the present teachings.
**Figure 14** depicts illustrative data according to the present teachings.
**Figures 15A-B** depict illustrative data according to the present teachings.
**Figures 16A-B** depict illustrative data according to the present teachings.
**Figure 17** depicts illustrative data according to the present teachings.
**Figures 18A-C** depict illustrative data according to the present teachings.
**Figure 19** depicts illustrative data according to the present teachings.
**Figure 20** depicts illustrative data according to the present teachings.

**DETAILED DESCRIPTION**

The practice of the present teachings will employ, unless otherwise indicated, conventional techniques of molecular biology (including recombinant techniques), microbiology, cell biology, biochemistry, and animal feed pelleting, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); Gene Transfer and Expression: A Laboratory Manual (Kriegler, 1990), and Fairfield, D. 1994. Chapter 10, Pelleting Cost Center. In Feed Manufacturing Technology IV. (McEllhiney, editor), American Feed Industry Association, Arlington, Va., pp. 110-139.

Unless defined otherwise herein, all technical and scientific terms used herein have the same meaning as commonly understood by one of ordinary skill in the art to which the present teachings belong. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present teachings.

Numeric ranges provided herein are inclusive of the numbers defining the range.

## *Definitions*

As used herein, the term "granule" refers to a particle which contains a core, an active agent, and at least one coating layer.

As used herein, the term "core" refers to the inner nucleus of a granule. The cores of the present teachings may be produced by a variety of fabrication techniques including: rotary atomization, wet granulation, dry granulation, spray drying, disc granulation, extrusion, pan coating, spheronization, drum granulation, fluid-bed agglomeration, high-shear granulation, fluid-bed spray coating, crystallization, precipitation, emulsion gelation, spinning disc atomization and other casting approaches, and prill processes. Such processes are known in the art and are described in US Pat. No. 4689297 and US Pat. No. 5324649 (fluid bed processing); EP656058B1 and US Pat. No. 454332 (extrusion process); US Pat. No. 6248706 (granulation, high-shear); and EP804532B1 and US Pat. No. 6534466 (combination processes utilizing a fluid bed core and mixer coating).

The core may include the enzyme, which may or may not be coated around a seed. Suitable cores for use in the present teachings are preferably a hydratable or porous material (i.e., a material which is dispersible or soluble in water) that is a feed grade material. The core material can either disperse in water (disintegrate when hydrated) or solubilize in water by going into a true aqueous solution. Clays (for example, the phyllosilicates bentonite, kaolin, montmorillonite, hectorite, saponite, beidellite, attapulgite, and stevensite), silicates, such as sand (sodium silicate), nonpareils and agglomerated potato starch or flour, or other starch granule sources such as wheat and corn cobs are considered dispersible. (Nonpareils are spherical particles made of a seed crystal that has been built onto and rounded into a spherical shape by binding layers of powder and solute to the seed crystal in a rotating spherical container. Nonpareils are typically made from a combination of a sugar such as sucrose, and a powder such as cornstarch.) The core may comprise a sodium chloride or sodium sulfate crystal, also referred to as a seed, or other inorganic salt crystal. In another example, the core comprises a sucrose crystal seed. Particles composed of inorganic salts and/or sugars and/or small organic molecules may be used as the cores of the present teachings. Suitable water soluble ingredients for incorporation into cores include: inorganic salts such as sodium chloride, ammonium sulfate, sodium sulfate, magnesium sulfate, zinc sulfate; or urea, citric acid, sugars such as sucrose, lactose and the like. Cores of the present teachings may further comprise one or more of the following: additional active agents, feed or food grade polymers, fillers, plasticizers, fibrous materials, extenders and other compounds known to be used in cores. Suitable polymers include polyvinyl alcohol (PVA), including partially and fully hydrolyzed PVA, polyethylene glycol, polyethylene oxide, polyvinyl pyrrolidine, and carbohydrate polymers (such as starch, amylose, amylopectin, alpha and beta-glucans, pectin, glycogen), including mixtures and derivatives thereof. Suitable fillers useful in the cores include inert materials used to add bulk and reduce cost, or used for the purpose of adjusting the intended enzyme activity in the finished granule. Examples of such fillers include, but are not limited to, water soluble agents such as salts, sugars and water dispersible agents such as clays, talc, silicates, cellulose and starches, and cellulose and starch derivatives. Suitable plasticizers useful in the cores of the present teachings are low molecular weight organic compounds and are highly specific to the polymer being plasticized. Examples include, but are not limited to, sugars (such as, glucose, fructose and sucrose), sugar alcohols (such as, sorbitol, xylitol and maltitol and other glycols), polar low molecular weight organic compounds, such as urea, or other known plasticizers such as water or feed grade plasticizers. Suitable fibrous materials useful in the cores of the present teachings include, but are not limited to: cellulose, and cellulose derivatives such as HPMC (hydroxy-propyl-methyl cellulose), CMC (carboxymethyl cellulose), HEC (hydroxy-ethyl cellulose). In another example particularly suitable for household cleaning applications, the core comprises a water-soluble or dispersible sugar or salt crystal or a non pareil. Those skilled in the art will recognize that, for feed and food applications, the cores (and any polymers, fillers, plasticizers, fibrous materials, and extenders), are acceptable for food and/or feed applications. For household cleaning applications, such a restriction need not apply.

Enzymes susceptible to problematic glycation include phytases and cellulases. Enzymes may include phytases, such as for example Finase L®, a phytase from Aspergillus sp., available from AB Enzymes, Darmstadt, Germany; Phyzyme™ XP, a phytase from E. Coli, available from DuPont Nutrition and Health, and other phytases from, for example, the following organisms: Trichoderma, Penicillium, Fusarium, Buttiauxella, Citrobacter, Enterobacter, Penicillium, Humicola, Bacillus, and Peniophora, as well as those phytases described in US patent applications 61/595,923 and 61/595,941, both filed February 12, 2012 . An example of a potential cellullase is Multifect® BGL, a cellulase (beta glucanase), available from DuPont Industrial Biosciences and other cellulases from species such as Aspergillus, Trichoderma, Penicillium, Humicola, Bacillus, Cellulomonas, Penicillium, Thermomonospore, Clostridium, and Hypocrea. The cellulases described in US20060193897A1 also may be used. Commercially available cellulases that find use in the present include, but are not limited to CELLUZYME®, CAREZYME® (Novozymes), and KAC-500(B)™ (Kao Corporation). Phytases and cellulases are enzymes frequently used for inclusion in animal feed and may find application in the present teachings. In some embodiments, chemically or genetically modified mutants may be included. Enzymes suitable for inclusion into detergents for household care applications are cellulases, phytases, and mixtures thereof.

As used herein, the term "enzyme matrix" refers to recovered enzyme arising from a fermentation, and may include additional components as processing aids including for example binders (e.g. PVA), anti-foam agents, surfactants (e.g. lutensol), starch, and sugars.

The term "coating layer" and "layer" as used herein are interchangeably. The first coating layer generally encapsulates the core in order to form a substantially continuous layer so that the core surface has few or no uncoated areas. Subsequent coating layers can encapsulate the growing granule to form one or more additional substantially continuous layer(s). Accordingly, as used herein, an "additional layer" refers to one or more coatings applied to a granule, and can contain any of a variety of materials readily available to one of routine skill in the art, including those materials described under "cores", as well as may be found in the relevant arts, including US Patent 7,018,821, US Patent 8,076,113, US Patent 4,106,991, US Patent 4,689,297, and US Patent 4,740,469.

As used herein, the term "resistant starch" refers to a starch that has been modified to reduce the production of glucose when treated with a glucoamylase. Specifically, a starch as used herein will be deemed "resistant starch" if less than 0.2 mM glucose is produced when 2% w/w of the starch of interest is incubated with *Trichoderma reesei* wild type glucoamylase for 40 minutes at 25C in 50 mM NaOAC (pH 4.5), by measuring total reducing ends with the BCA method using a glucose calibration curve.

As used herein, the term "enzyme susceptible to problematic glycation" refers to an enzyme which when subjected to the following assay shows at least a 20% reduction in activity relative to the control. An enzyme solution is obtained, 1% w/w glucose is added to it, the resulting mixture is lyophilized, and the resulting dry solid is exposed to 50C/70% RH for 5 days, redissolved, and the activity of this sample is measured and compared to a control sample exposed to 50C/to% RH but without the added glucose. If the enzyme with glucose added loses more than 20% activity than the control, it is "susceptible to problematic glycation." In some embodiments, at least a 30%, at least a 40%, at least a 50%, at least a 60%, at least a 70%, at least an 80%, or at least a 90% reduction is produced.

As used herein, the term "problematic sugar" generally refers to glucose, fructose, and sucrose.

As used herein, the term "glycatable amino acid residue" refers to an amino acid in a protein that is susceptible of undergoing covalent addition of a sugar. Examples include lysine and arginine, though it will be appreciated that other non-naturally occurring amino acids, if found in a protein and susceptible of undergoing covalent addition of a sugar, would fall within the scope of the present teachings.

As used herein, the term "one diavolume" equals the amount of filtrate recovered in comparison to an equal volume of retentate held at constant volume while adding fresh water or buffer during a process of continuous diafiltration or dialysis.

As used herein, the term "Percent sequence identity" means that a variant has at least a certain percentage of amino acid residues identical to a protein of interest, when aligned using the CLUSTAL W algorithm with default parameters. See Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |

(continued)

| | |
|---|---|
| Toggle Residue specific penalties: | ON |
| Toggle hydrophilic penalties: | ON |
| Toggle end gap separation penalty | OFF |

Deletions are counted as non-identical residues, compared to a reference sequence. Deletions occurring at either terminus are included. For example, a variant with six amino acid deletions of the C-terminus of a mature polypeptide 612 residue molecule would have a percent sequence identity of 99% (606/612 identical residues $\times$ 100, rounded to the nearest whole number) relative to the mature polypeptide. Such a variant would be encompassed by a variant having "at least 99% sequence identity" to a protein of interest.

**Exemplary Embodiments**

Enzymes susceptible to problematic glycation are identified. Thereafter, the enzyme is made so as to ensure glycation is reduced relative to an enzyme made without such ensurances, so as to improve enzyme stability relative thereto. Various approaches can be undertaken so as to ensure glycation is reduced, as provided in the present examples and discussed in the included claims, and include diafiltration, engineering out problematic amino acid residues, heating to inactivate problematic enzymes remaining from a fermentation, formulating without problematic sugars, formulating using resistant starches, and combinations thereof.

Provided herein is a method of improving enzyme stability comprising; making an enzyme in a fermentation, wherein the fermentation comprises a fermentation medium comprising problematic sugars and hydrolyzable starch, and wherein the enzyme comprises at least one glycatable amino acid residue and is an enzyme susceptible to problematic glycation; recovering the enzyme; and, removing sufficient amounts of the problematic sugars to improve enzyme stability relative to a similarly fermented enzyme lacking the removing.

In some embodiments, the removing comprises diafiltering an ultrafiltrate concentrate to remove the problematic sugars to form an enzyme concentrate. In some embodiments, the diafiltering comprises at least two, at least three, or at least four, diavolumes, such that the sugars content of the enzyme concentrate is reduced to less than 0.1% w/w. In some embodiments, the removing comprises heating the enzyme after the fermentation at 50C for at least 1, 2, 4, 8, or 12 hours to inactivate any starch-hydrolyzing enzymes present. In some embodiments, the removing comprises a previous genetic manipulation, wherein at least one active site lysine residue was removed from the polypeptide. In some embodiments, the active lysine residue is K131 and/or K26, and the enzyme is wild type *Buttauxella sp.* Phytase, or a molecule 90%, 95%, 97%, 98%, or 99% identical thereto. In some embodiments, the active lysine residue is K131 and/or K26, and the enzyme is BP17.

In some embodiments, the present teachings provide an engineered phytase wherein an amino acid residue at position 131 and/or 26 relative to wild-type buttiauxella phytase is not lysine. In some embodiments, the position 131 residue is arginine.

Also provided is a method of improving an enzyme's stability comprising; determining the existence of at least one glycatable amino acid residue in an enzyme; determining whether the enzyme is susceptible to problematic glycation; making the enzyme so as to ensure glycation is reduced relative to an enzyme made without such ensurances, so as to improve enzyme stability relative thereto. In some embodiments, the ensurances comprise mutating at least one lysine residue. In some embodiments, the ensurances comprise treating the enzyme with heat. In some embodiments, the ensurances comprise diafiltering the enzyme. Ensurances may comprise combining various approaches, for example, mutating and heat treatment, or, mutating and formulating with resistant starch, or, mutating and diafiltering, or, heat treatment and diafiltering, or, mutating and diafiltering, or, mutating and heat treatment and diafiltering, or, in other combinations as will be appreciated by one of ordinary skill in the art exercising routine combinatorial logic.

Also disclosed herein is an enzyme granule comprising an enzyme susceptible to problematic glycation, and, a resistant starch. The enzyme granule may comprise a) a sodium sulfate seed, b) an enzyme layer comprising the enzyme susceptible to problematic glycation, the resistant starch, sodium phytate, and rapeseed oil, c) a sodium sulfate layer, and, d) an external layer comprising PVA and talc. The enzyme present in the enzyme granule may be a phytase. The enzyme may be wild type *Buttiauxella sp.* The enzyme may be BP17, or an amino acid sequence 90%, 95%, 97%, or 99% identical to it. The enzyme may be a cellulase. The enzyme may be the cellulase exemplified herein, or an amino acid sequence 90%, 95%, 97%, or 99% identical to it.

The enzymes and compositions of the present teachings will found application of any of a variety of fields for example biomass processing (e.g.-cellulases) and animal feed (e.g.-phytases). The enzymes of the present teachings can be formulated as solids (eg-enzyme granules), or as liquids (e.g.-liquids containing suitable amounts of for examples sorbitol and salt), or indeed in any form that one of skill in the art would recognize their applicability.

The invention can be further understood by reference to the following examples, which is provided by way of illustration and not meant to be limiting.

**Example 1**

**Activity Loss Data**

To explore the effect of time on phytase stability, a BP17 phytase was expressed and purified to form a UFC. BP17 is an enzyme variant of a *Buttiauxella sp.* phytase. The sequence for BP17 (excluding signal peptide) is as follows, and is described in PCT/US2013/0244415.

SEQ ID NO: 1

NDTPASGYQVEKVVILSRHGVRAPTKMTQTMRDVTPNTWPEWPVKLGYITPRGEHLI

SLMGGFYRQKFQQQGILSQGSCPTPNSIYVWTDVAQRTLKTGEAFLAGLAPQCGLTIH

HQQNLEKADPLFHPVKAGICSMDKTQVQQAVEKEAQTPIDNLNQHYIPSLALMNTTLN

FSKSPWCQKHSADKSCDLGLSMPSKLSIKDNGNEVSLDGAIGLSSTLAEIFLLEYAQG

MPQAAWGNIHSEQEWALLLKLHNVYFDLMERTPYIARHKGTPLLQAISNALNPNATES

KLPDISPDNKILFIAGHDTNIANIAGMLNMRWTLPGQPDNTPPGGALVFERLADKSGKQ

YVSVSMVYQTLEQLRSQTPLSLNQPAGSVQLKIPGCNDQTAEGYCPLSTFTRVVSQS

VEPGCQLQ

The fermentation of proteins including BP17 phytase is described in WO2004035070A1 and US7713725B2. After fermentation, the fermentate is passed through a rotary drum vacuum filtration unit which can be precoated with diatomaceous earth to remove the cells from the fermentate. Next, the cell free fermentate is concentrated by passing it through a 10,000 molecular weight cutoff ultrafiltration membrane removing enough permeate until the final activity is between 30,000 and 80,000 FTU/g.

The recovered fermentation concentrate is called "ultrafiltered concentrate" or UFC and contains the phytase protein along with other proteins, peptides, polysaccharides and sugars produced during the fermentation as byproducts. The UFC is combined with 0.3% Potassium Sorbate and 0.3% Sodium Benzoate to serve as a preservative prior to granulating the material. To make a 2000 gram batch of Axtra Phytase TPT®, 380 g of Phytase UFC having an activity of 74,250 units/g and a solids content of 25.6% is combined with 190 g of water, 70.6 g of sucrose, 152.9 g of corn starch, 17.6 g of rapeseed oil and 11.8 g of sodium phytate and this mixture is sprayed onto 722 g of sodium sulfate seeds which have been charged to a Vector VFC-1 fluid bed granulator. For this first spray coating, the inlet temperature was 60°C, the outlet temperature was 40°C, the spray rate was 11 g/min, the atomization air pressure was 38 psi and the fluidization air velocity was 75 cfm. Next a solution of 870 g of sodium sulfate dissolved in 2475 g of water is sprayed onto the granule using an inlet temperature was 68°C, the outlet temperature was 47°C, the spray rate was 25 g/min, the atomization air pressure was 38 psi and the fluidization air velocity was 75 cfm. The final coating was prepared by dissolving 70.59 g of polyvinyl alcohol (Mowial 5-88®) in 965 g of water with 141 g of talc at 90°C for 30 minutes. After this solution is allowed to cool back to room temperature, it is sprayed onto the granule using an inlet temperature of 70°C, an outlet temperature of 51°C, a spray rate of 11 g/min, an atomization air pressure of 40 psi and a fluidization air velocity of 75 cfm. Then, 20 gram samples of Axtra Phytase TPT® enzyme granules were placed in a 200 ml closed polyethylene container stored at 4°C, 25°C and 40°C for 12 months. At regular time intervals, phytase activity was determined by both the para-nitrophenyl phosphate (pNPP) assay and the molybdenum vanadat (Mo-V) assay following dissolution with0.25 M Sodium Acetate Buffer, pH 5.5. In the *p*NPP assay, a synthetic substrate, para-nitrophenyl phosphate, is employed. Upon dephosphorylation with phytase and basification of the reaction mixture, the para-nitrophenylate anion is formed, which absorbs at 405 nm. In the Mo-V assay, sodium phytate is used as a substrate. Phytate desphosphorylation is coupled to absorbance at 415 nm upon treatment with an acidic molybdenum-vanadate cocktail. The *p*NPP assay is a generic phosphatase assay, while the Mo-V assay is specific to phytase

The pNPP Assay can be performed on an automated Konelab® robot as follows. All samples were diluted in 0.25 M acetate pH 5.5 buffer (Dissolve 30.02 g sodium acetate trihydrate (or 18.10 g sodium acetate anhydrous), 1.76 g concentrated acetic acid (= 1.677 ml) and 0.147 g calcium chloride dihydrate in 900 ml Milli-Q water. Adjust to pH 5.5 with 4 M acetic acid (22.9 ml concentrated acetic acid in 100 ml Milli-Q water) or 100% acetic acid. Add 1 ml TWEEN 20 and adjust volume to 1000 ml with Milli-Q water.). Once diluted samples were loaded into the Konelab, 7 $\mu$l of sample was combined with 84 $\mu$l of pNPP substrate solution (4-nitrophenyl phosphate (Npp) di(-tris) salt (FW 461.4) (Sigma N-3254) Weigh 0.09228 g 4-Nitrophenyl phosphate and dissolve in 10 mL Assay Buffer) and allowed to incubate at 30 °C for 5 minutes. Next, 105 $\mu$l of 200 mM borate stop solution (Dissolve 6.183g boric acid in 400 mL MilliQ water. Adjust pH to 10.2 with 1N NaOH. Q.S. to 500 mL with MilliQ water.) is added to the sample and substrate to quench the reaction for 2 minutes. The absorbance at 405 nm is read and converted to activity units of FTU/g through the use of a calibration curve containing phytase standards at 0, 2.5, 13.0 and 27.0 FTU/g.

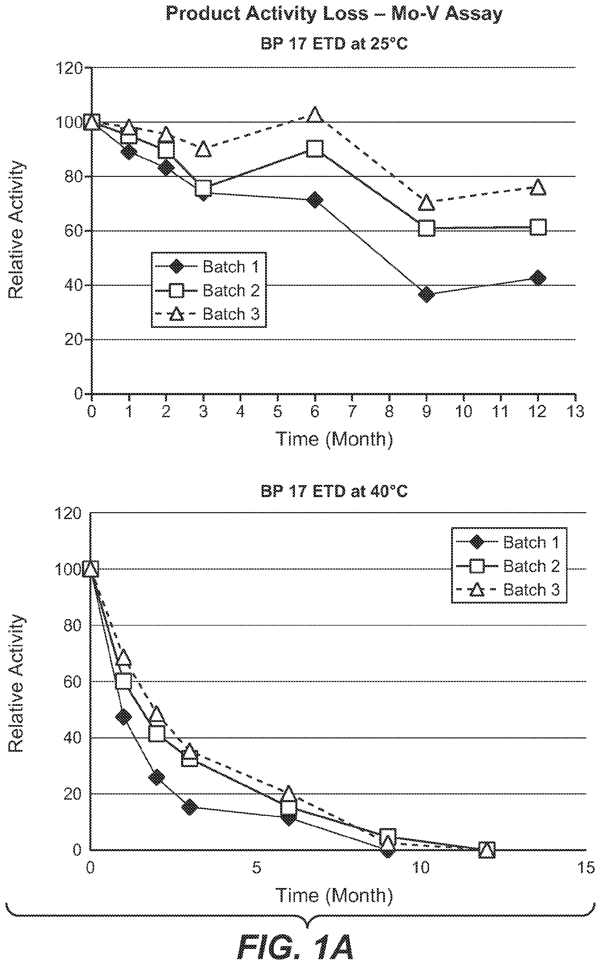

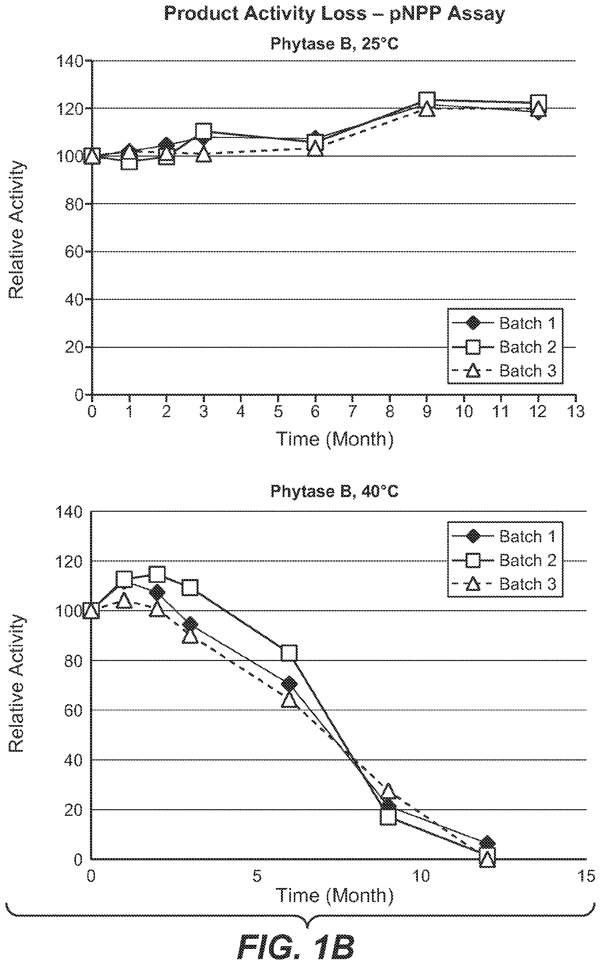

The graphs in Figures 1A and 1B summarize the activity loss suffered by TPT Phytase during storage. At 25°C granule suffer a 30 - 70% Mo-V activity (sodium phytate substrate) loss over 12 months. No activity is lost in the pNPP assay (synthetic substrate) for the same samples over the same time period. At 40°C TPT phytase loses 95 - 100% of its Mo-V activity over 6 months and only 20 - 40% of its pNPP activity for the same samples over 6 months.

It was hypothesized that the differential loss of activity between the pNPP and Mo-V assay is due to glycation of lysine residues near the phytase active site which could alter substrate specificity towards the larger, natural phytic acid substrate but not for the smaller synthetic para-nitrophenyl phosphate (pNPP) substrate.

## Example 2

### Maillard Reaction and Browning

To test the role of glycation, the Maillard browning reaction was monitored visually by depositing a solution BP17 ETD phytase UFC onto a circle of filter paper, drying the enzyme and then placing the enzyme in an environmental chamber at 50°C and 70% relative humidity for 5 to 12 days.

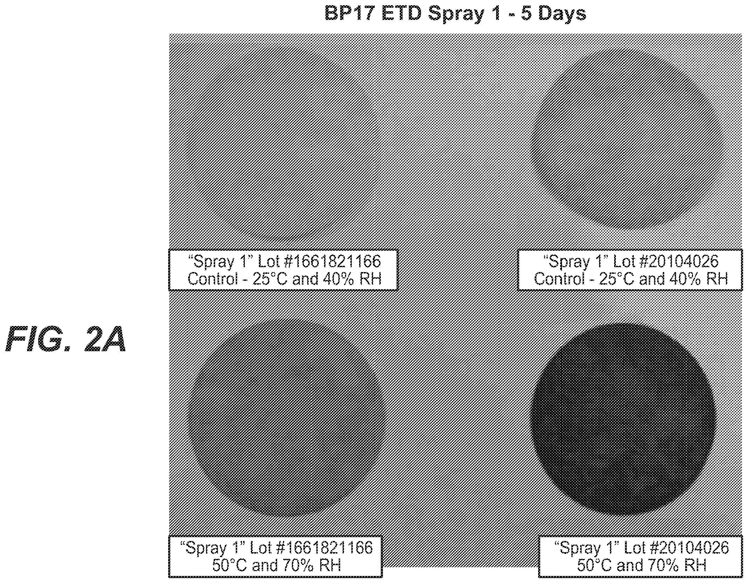

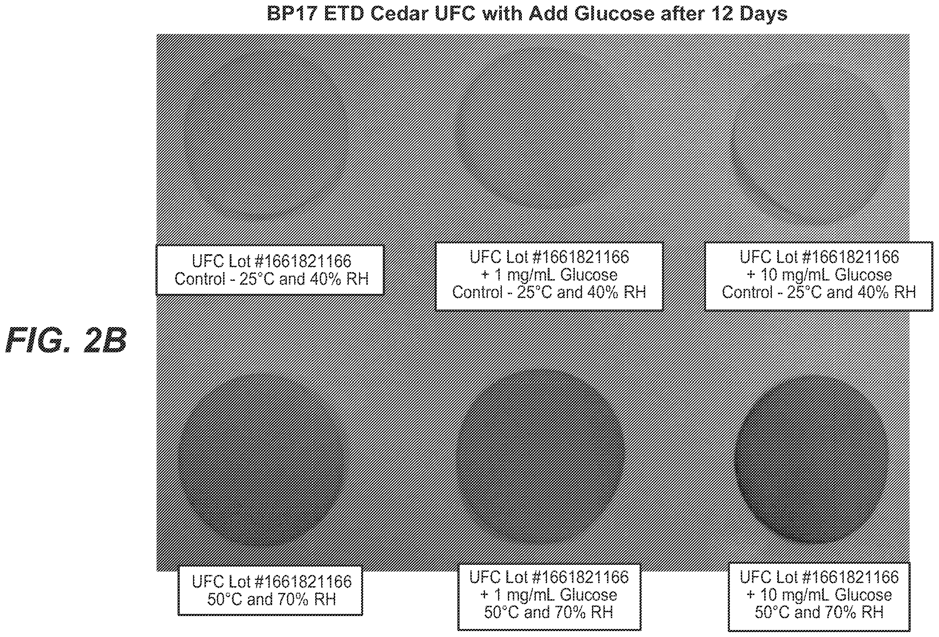

Figure 2A shows darkening of the dry phytase enzyme spray 1 (composed of 32% enzyme solids, 41% corn starch, 19% sucrose, 3% sodium phytate and 5% rapeseed oil) stored at 50°C and 70% relative humidity for 5 days for two different lots of enzyme UFC. Figure 2B shows one lot of phytase UFC with varying levels of added glucose stored at both 25°C/40% RH and 50°C/70% RH for 12 days. It can be seen that increasing levels of glucose, leads to increasing levels of browning.

Diafiltration of BP17 ETD UFC was performed using a 10K molecular weight cutoff membrane to filter away the permeate of the UFC (which contains all of the material less than 10K Daltons). Diafiltration was performed on a Amicon stirred cell (Amicon Model # 8400) fitted with a 10K Dalton molecular weight cutoff Polyethersulfone membrane (Sartorius No. 14639-76-----D) on top of a 10K Dalton molecular weight cutoff regenerated cellulose membrane (Millipore Cat. # PLGC07610). By performing dialfiltration to generate 3 times the original volume of the starting UFC in permeate (3 diavolumes), approximately 96% of all reducing sugars including glucose are removed from the UFC, greatly reducing the tendency for this UFC to undergo glycation or the Maillard reaction. The percent of permeable molecules removed during diafiltration can be described by the following equation:

$$\{1 - [e^{(V * T)}]^{-1}\} \times 100$$

where V is the number of diavolumes diafiltered and T is the transmission coefficient through the membrane. When we assume T = 1 (100% transmission) and V = 3 (3 diavolumes), them the percent permeable molecules diafiltered is (1 - $e^{-3}$) x 100 or 95%.

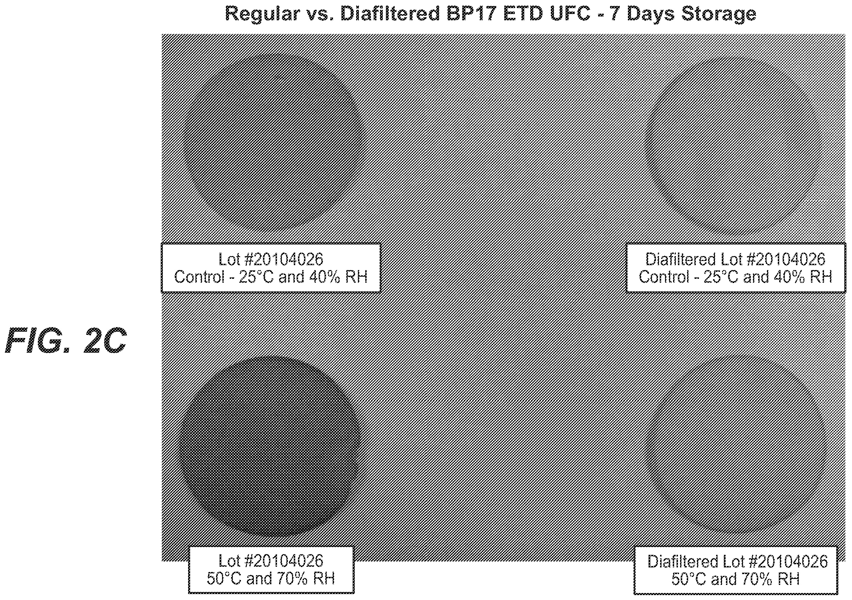

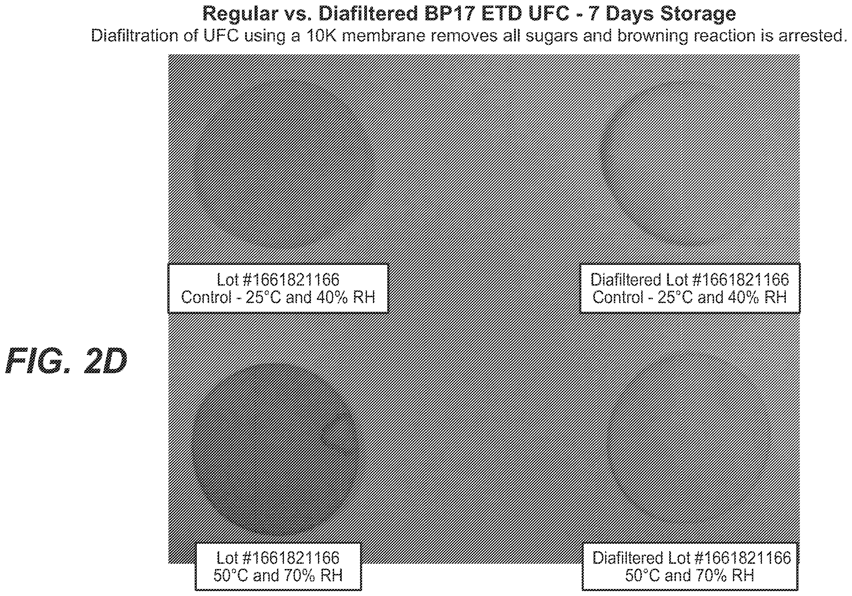

Figure 2C shows the effect of diafiltering BP17 ETD UFC on the browning reaction after 7 days of storage at 50°C and 70% relative humidity. It can be seen that the UFC shows almost no browning after diafiltering the enzyme UFC 3 diavolumes through a 10K membrane. Figure 2D shows the effect of diafiltering a second lot of BP17 ETD UFC on the browning reaction after 7 days of storage at 50°C and 70% relative humidity. It can be seen that the UFC shows almost no browning after diafiltering the enzyme UFC 3 diavolumes through a 10K membrane.

## Example 3

### Intrinsic Fluorescence

As proteins undergo the Maillard reaction they will form glycation products, which undergo further chemical reaction to form fluorescent heterocyclic products. The formation of these advanced glycation products can be monitored by measuring the intensity of their fluorescent emission signal. Approximately 0.15 g granule was weighed and mixed with 1.5 ml dilution buffer (100 mM NaOAc, pH 5.5), and allowed to dissolve for 20 min at room temperature. The granules were composed approximately 37% of a 200 - 300 $\mu$m core (a seed of sodium sulfate) coated with an enzyme layer composed of either (A) 2% polyvinyl alcohol (Mowial® 5-88), 0.50% sodium phytate and approximately 4% phytase enzyme solids or, (B) 3% sucrose, 6.5% corn starch, 0.50% sodium phytate and approximately 4% phytase enzyme solids. On top of the enzyme layer is a 40% layer of sodium sulfate followed by a final coating layer composed of 3% polyvinyl alcohol and 6% Talc. The sample was centrifuged at 3000 rpm for 10 min and filtered through 0.45 um filters. Zeba desalt spin columns (10 ml columns) were equilibrated 4 X 5 ml 20 mM sodium borate buffer at pH 8.5. One milliliter of the sample was added to each column with 0.2 ml of borate buffer to aid elution by centrifugation. The eluted sample was then placed into a microtiter plate and the fluorescence spectrum was monitored using a 335 nm excitation wavelength.

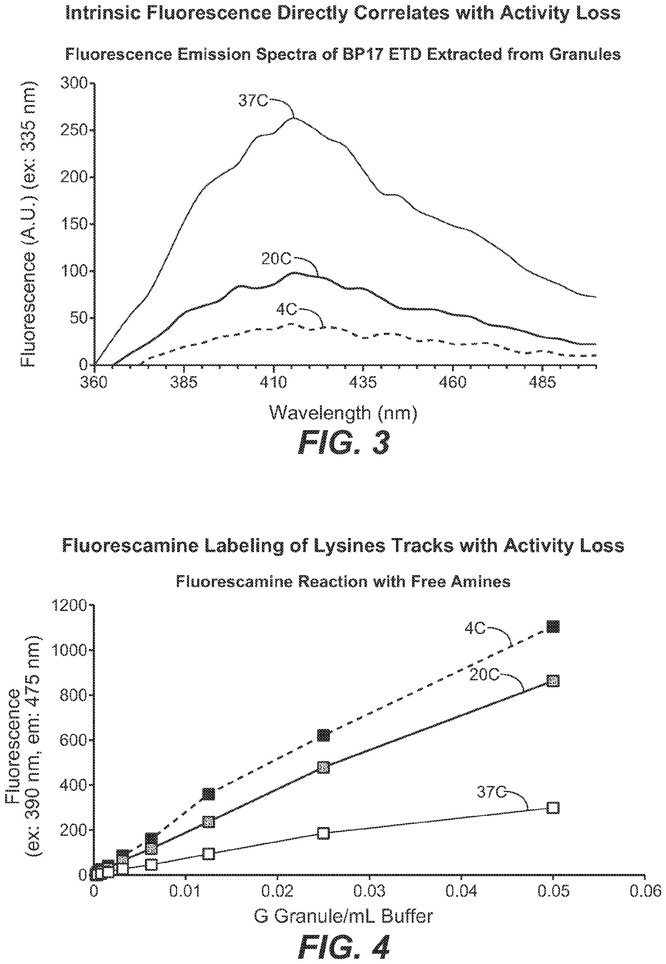

Figure 3 shows the fluorescence emission spectrum resulting from excitation at 335 nm for phytase extracted from TPT granules stored at 4°C, 25°C and 37°C for 12 months. The increase in fluorescence tracks with storage temperature. Since higher storage temperatures accelerate the rate of glycation and higher levels of glycation will lead to higher levels of advanced glycation end products (AGE's) and since the presence of these AGE's is responsible for the intrinsic fluorescence, it is concluded that higher levels of intrinsic fluorescence is also correlated to loss of phytase activity since higher levels of glycation of phytase is directly correlated to loss of phytase activity.

## Example 4

### Fluorescamine Assay

Similarly treated samples used in the Intrinsic Fluorescence measurements were used in the Fluorescamine Assay. Fluorescamine was used to directly measure the level of glycated lysine groups in phytase. Fluorescamine reacts with unmodified (non-glycated) lysine groups and becomes fluorescent after reaction.

Fluorescamine (8 mg) was mixed with DMSO (50 ml) to make a solution at a final concentration 0.16 mg/ml. Nine 2-fold serial dilutions of the desalted samples were prepared on a 96 well microtiter plate. Sample quantities of 75 ul were transferred to black fluorescent plates in duplicate. One set was combined with 18 ul of the DMSO/fluorescamine solution and the other was combined with 18 ul of DMSO alone. The solutions are allowed to react at room temperature for 5 minutes on shaker plate with a cover. The samples were excited at 390 nm and emission was monitored at 475 nm. Fluorescent emission values without fluorescamine were subtracted from those with fluorescamine and plotted. Figure 4 shows that the fluorescence emission intensity increased with concentration of phytase, but decreased with increasing temperature. The latter suggests an increase in glycated lysine groups as storage temperature is increased.

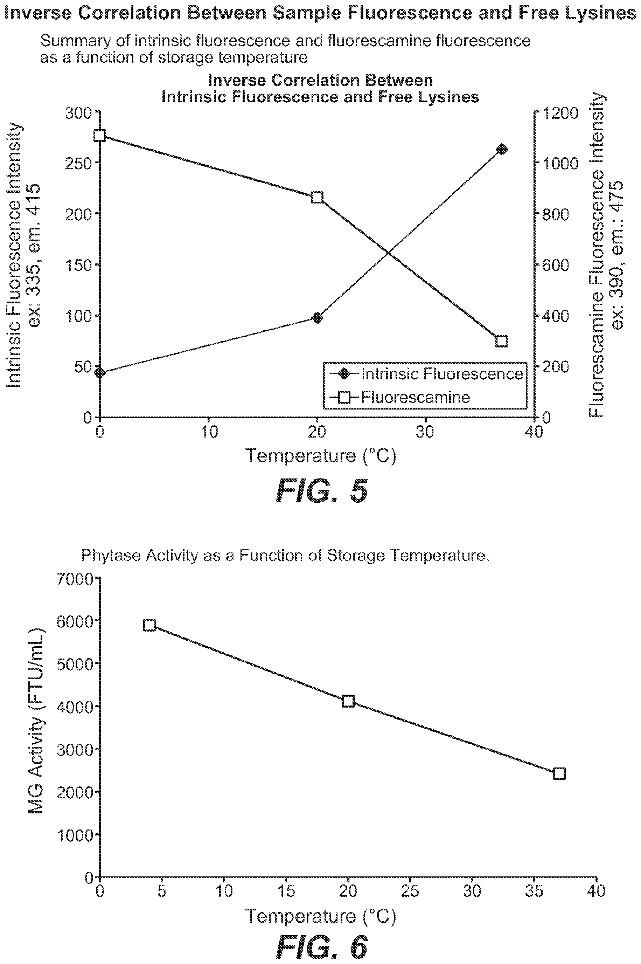

Figure 5 summarizes the effect of incubation temperature on intrinsic fluorescence intensity and fluorescamine-derived fluorescence intensity. Intrinsic fluorescence intensity at 415 nm corresponds with the formation of Maillard products. Fluorescamine-derived fluorescence intensity at 475 nm is due to the reaction of free amino groups with fluorescamine and therefore, represents a lack of Maillard products. Data were obtained from the experiments noted above and plotted on the same graph to illustrate how Maillard products increase with incubation temperature (intrinsic fluorescence), while free lysines decrease (reflected in the diminished fluorescamine-derived signal). Figure 6 depicts the measured activities for the same sample incubated at the various temperatures. The activity assay was performed with the malachite green reagent, which couples absorbance at 620 nm with phosphate released from a phytate substrate. The attenuation of activity at increased temperature shown in Figure 6 tracks with increase in Maillard products displayed in Figure 5.

## Example 5

### Glvcation Analysis via Mass Spectrometry

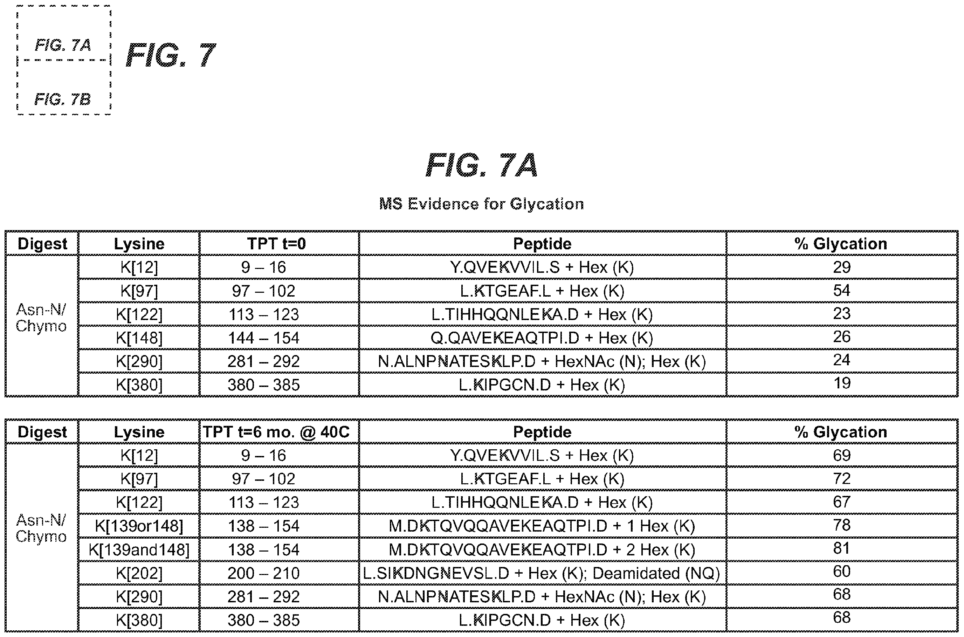

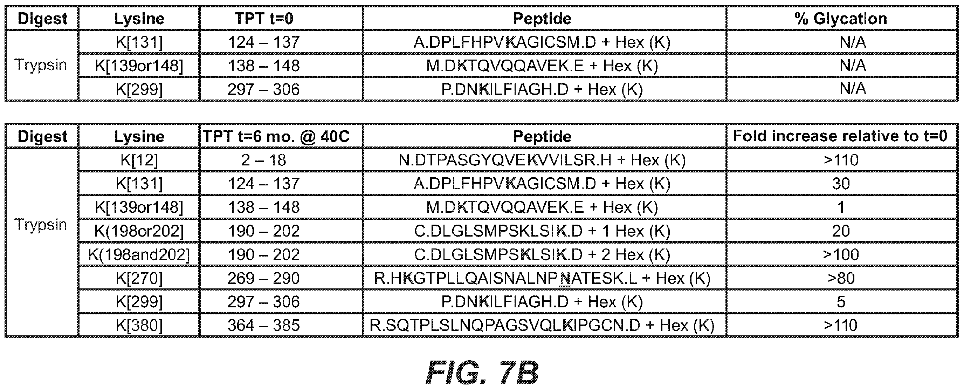

Mass spectrometry evidence for glycation of phytase is shown in Figures 7A-B. These data were obtained from an Asp-N endoproteinase/chymotrypsin and trypsin digests with database searches for hexose (i.e. glycation) modification on lysine residues. For the Asp-N/chymotrypsin digest, quantitation was established by obtaining the ratio of glycated vs. non-glycated peptides. It is important to note the quantification numbers are only approximate as glycation of a lysine residue is expected to decrease its ionization efficiency. In the AspN/chymotrypsin digests at time 0, between 19 and 54% of the lysine groups on phytase are glycated. After storage for 6 months at 40C, the percentage of glycation increases for all lysines increases to between 67 and 81%. Trypsin digests were also performed, but could not be quantified in the same manner due to the fact that this enzyme cleaves at lysine and arginine residues. Accordingly, the fold increase in modification relative to t=0 for the trypsin digest was determined by normalization to an internal standard peptide In the trypsin digest, glycation increase is observed on several lysines by factors ranging up to ~110-fold.

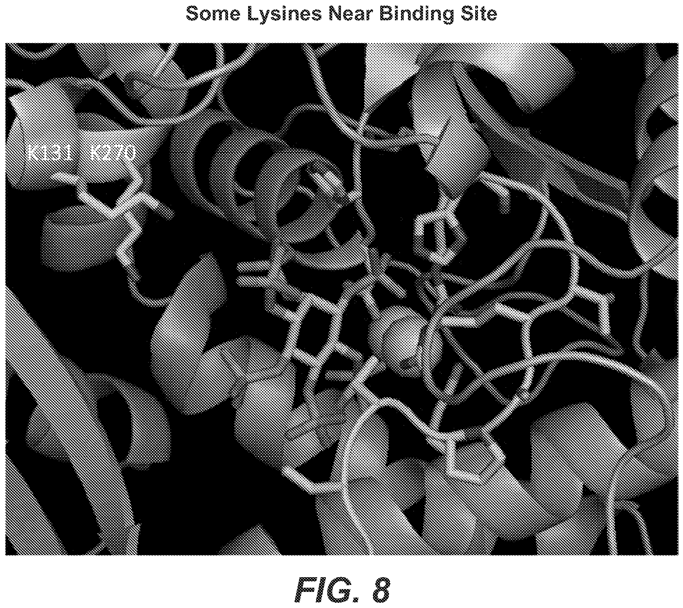

In Figure 8, the two lysines, K131 and K270, located closest to the active site of phytase are highlighted. Without being bound by theory, it was hypothesized that it is the glycation of one, both, or other lysines near the active site which is responsible for the reduction in activity of phytase towards its natural substrate, phytate while retaining nearly all of its activity against the much smaller synthetic substrate para-nitrophenylphosphate (pNPP).

## Example 6

### Induced Glycation in Lyophilized Samples of Purified BP17 ETD

2 mg/ml of BP17 ETD phytase was purified via high ion exchange chromatography and combined with 0 to 0.0625 M of glucose or fructose in solution. These solutions were lyophilized to produce a dry solid and this lyophilized material was incubated at 50°C and 70% relative humidity for 6 days to induce glycation or the Maillard reaction of the phytase with the added reducing sugar.

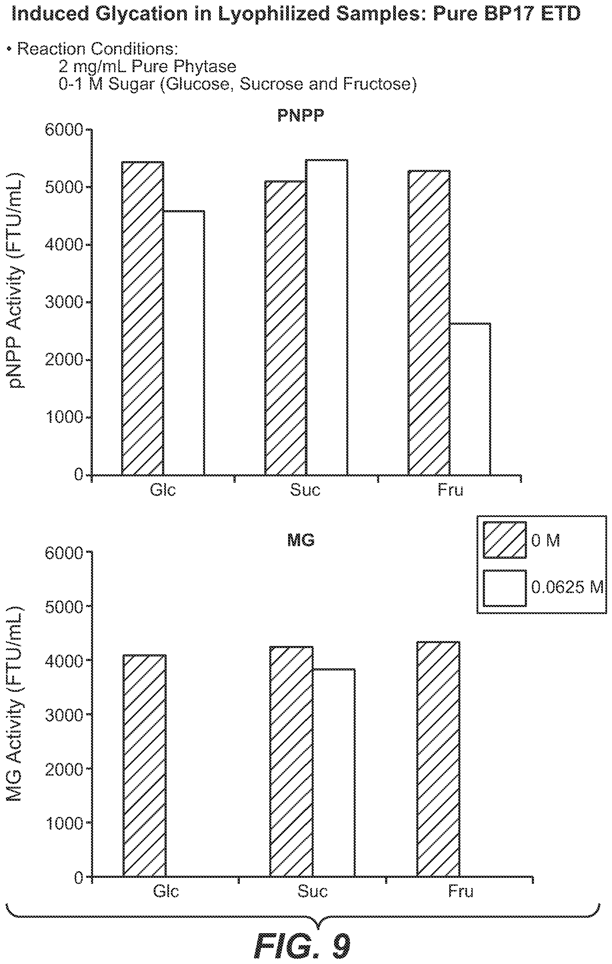

Figure 9 shows the effect of induced glycation from glucose and fructose after incubating 0.0625 M of the sugar with phytase at 50°C and 70% relative humidity for 6 days. It can be seen that for glucose the pNPP activity is reduced from 5500 FTU/ml to 4500 FTU/ml while the activity on phytate measured via the MG assay is reduced from 4000 FTU/ml to 0 FTU/ml. Likewise for fructose the activity is reduced from 5200 FTU/ml to 2700 FTU/ml towards pNPP and from 4100 FTU/ml to 0 FTU/ml for fructose. As expected, incubation with the non-reducing sugar, sucrose, did not result in any significant loss of either pNPP or phytase activity. Similar results were obtained for phytase from UFC.

## Example 7

### Heat Treatment of BP17 ETD Phytase UFC to Remove Background Activities

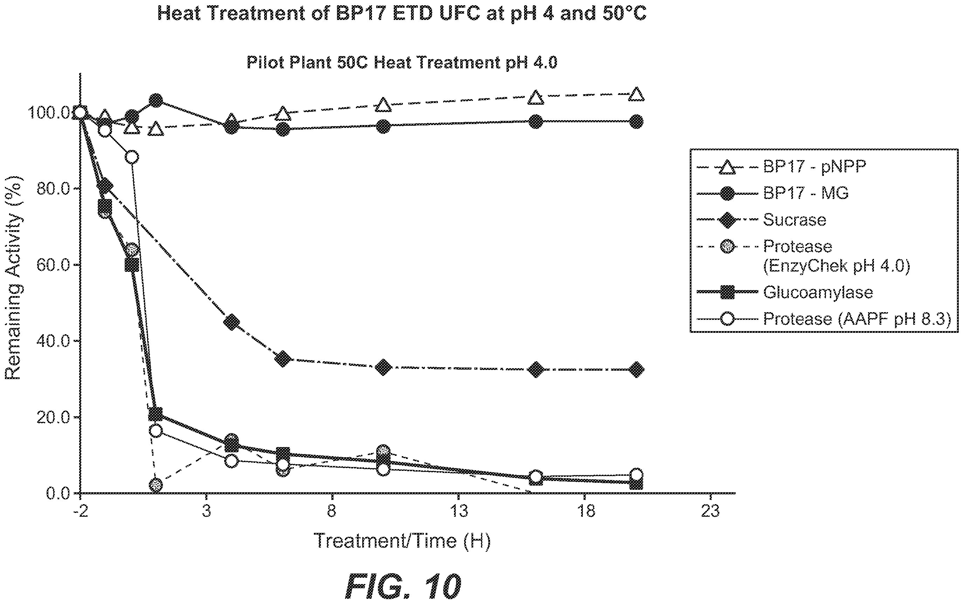

Heat treatment of the BP17 ETD phytase UFC is done to remove background sucrase and glucoamylase activity which can generate glucose during storage from sucrose and starch in the formulation, respectively. Figure 10 shows an example of a heat treatment process and the corresponding reduction in measured background activities with time of heating. Phytase UFC is heated to 50°C for 4 to 20 hours at pH 4.0 and this causes glucoamylase activity reduction between 90 and 95% and sucrase activity reduction of about 65%. Protease activity is also reduced by 90 - 95% but this activity is not believed to be responsible for glucose generation or glycation of the phytase.

## Example 8

### Accelerated Storage Stability

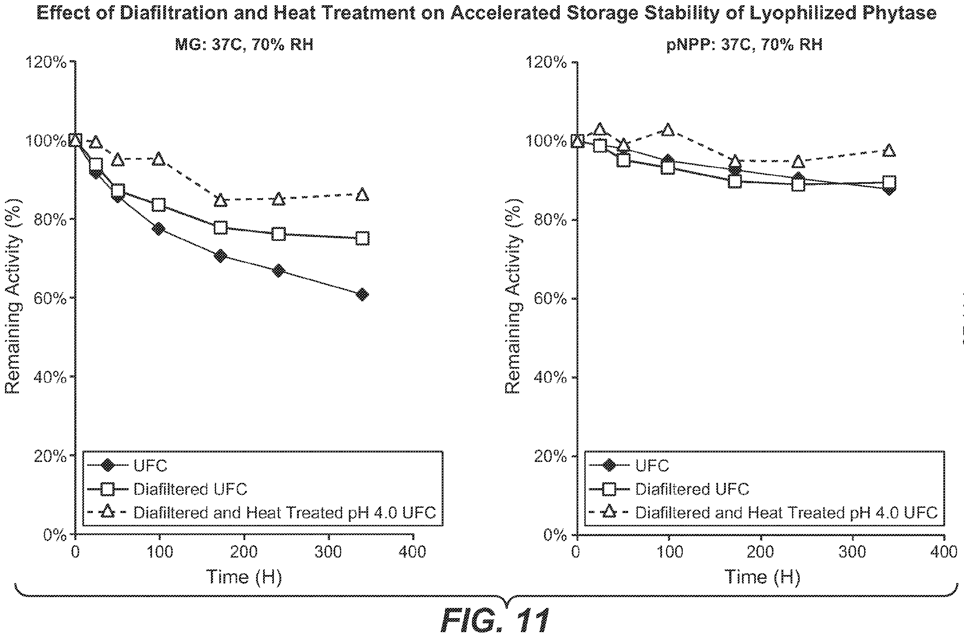

Figure 11 shows the effect of both diafiltration and heat treatment (50C for 16 hours) on the retained activity on lyophilized UFC. Diafiltration was carried out with 3 kg of Phytase UFC. The starting UFC had a pH of 4.0 and conductivity of -2.7 S/cm. A Millipore unit with 10kDa Polyethersulphone (PES) Millipore membrane was used for this experiment. The UFC was diafiltered with 3 diavolumes of process water in batch mode at a temperature of 10-15°C. Batch mode here refers to a technique in which 1 diavolume of water is added upfront to the UFC followed by collection of 1 diavolume of UF permeate. This process is repeated 3 times in order to achieve 3 diavolumes of diafiltration. At the end of the diafiltration, if the pH of the UFC has drifted > 4.5, 20% acetic acid is used to adjust the pH down to 4.0.

It can be seen that both diafiltration and heat treatment improve the storage stability of the dry phytase towards its natural substrate phytate (MG Assay) while only heat treatment improves the storage stability of the dry phytase towards its synthetic pNPP substrate. This is most likely because diafiltration is primarily removing glucose and other reducing sugars and therefore preventing glycation of the phytase . In contrast, heat treatment is primarily deactivating the background enzymes protease, glucoamylase and sucrase and since no sucrose or starch is present in these UFC's, the heat treatment is primarily preventing proteolysis which affects activity towards both the synthetic and real substrate.

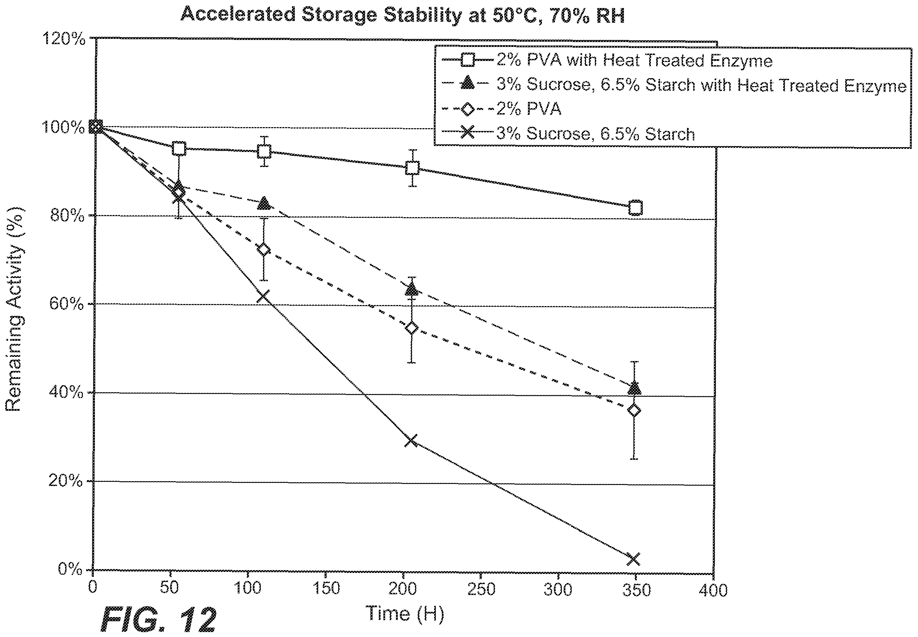

Figure 12 shows the phytase activity remaining for granules having four different enzyme matrix compositions stored at 50°C and 70% relative humidity. The granules were compositionally similar to those described in Example 3. For the granule made with 3% sucrose and 6.5% corn starch in the enzyme matrix with untreated enzyme concentrate, it can be seen that only 3% activity is remaining after 350 hours of storage. When 2% PVA is substituted for the sucrose and corn starch in the enzyme matrix, thus getting rid of all internal sources of glucose generation, it can be seen that the activity after 350 hours of storage is now increased to 37%. Similarly, when the sucrose/starch enzyme matrix based granule is made with heat treated enzyme, thus deactivating the enzymes responsible for glucose formation in the enzyme matrix, the retained activity after 350 hours is also increased, this time to 42%. Finally, if the granule is made with both 2% PVA in the enzyme matrix and heat treated enzyme concentrate, the final retained activity after 350 hours of storage is 82%. This is believed due to the deactivation of a protease which occurs during the heat treatment process combined with the removal the glucose forming sucrose and starch substrates.

**Example 9**

**DXST**

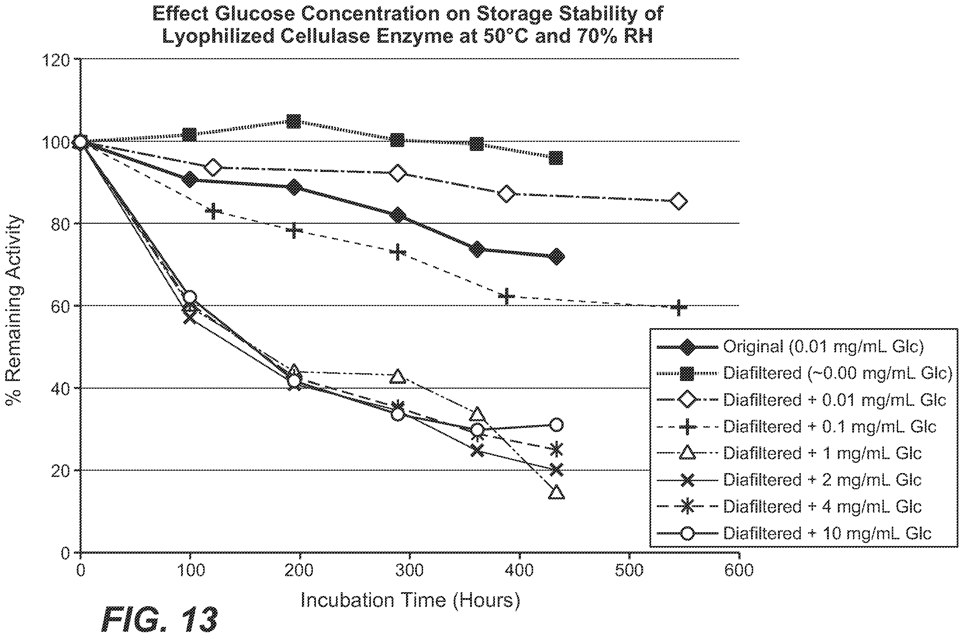

Figure 13 shows the stability of various lyophilized cellulase enzyme UFC's at 50°C and 70% RH as a function of glucose concentration. DXST was expressed in Trichoderma resei, its sequence is provided below.

## SEQ ID NO:2

MRSSPVLRTALAAALPLAALAADGKSTRYWDCCKPSCSWPGKASVNQPVFACSANF

QRISDPNVKSGCDGGSAYACADQTPWAVNDNFSYGFAATSISGGNEASWCCGCYEL

TFTSGPVAGKTMVVQSTSTGGDLGTNHFDLAMPGGGVGIFDGCSPQFGGLAGDRYG

GVSSRSQCDSFPAALKPGCYWRFDWFKNADNPTFTFRQVQCPSELVARTGCRRND

DGNFPVFTPPSGGQSSSSSSSSSAKPTSTSTSTTSTKATSTTSTASSQTSSSTGGGC

AAQRWAQCGGIGFSGCTTCVSGTTCNKQNDWYSQCL

The cellulase is an endo-beta-D-1,4-glucanase from Staphylotrichum coccosporum gi|197267671 (short name STCE1). It can be seen that the original UFC containing about 0.01 mg/ml glucose shows about a 30% loss of activity over 431 hours while the lyophilized UFC shows no loss of activity over the same time period. Adding glucose back to the lyphilized UFC causes the celllulase to become unstable again causing as much as 80% loss of activity over 450 hours at glucose concentrations of 1 mg/ml and higher. Interestingly, adding 0.01 mg/ml glucose back to the diafiltered UFC does not cause as much activity loss as the original UFC which contains 0.01 mg/ml glucose. This can be explained by the hypothesis that glucose may not be the only reducing sugar present in the original UFC.

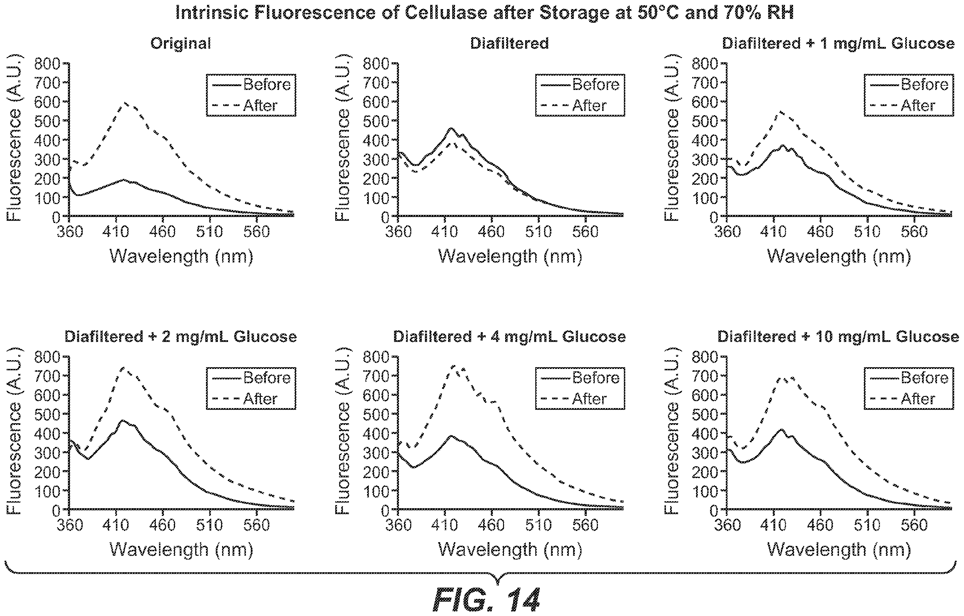

Figure 14 shows the intrinsic fluorescence of various lyophilized cellulase UFC's (similarly treated samples from Figure 13) after storage at 50°C and 70% RH for 431 hours. It can be seen that the original cellulase UFC shows a 300% increase in intrinsic fluorescence while the lyophilized cellulase shows only a 20% increase. Diafiltered cellulase UFC with added glucose shows progressively greater levels of intrinsic fluorescence showing up to a 150% increase in intrinsic fluorescence at the highest level of added glucose.

Without being bound by theory, it is hypothesized that at least one of the lysines near the active site include K25, K34, K42 and K65 may be responsible for this loss.

**Example 10-Protein Engineering Studies**

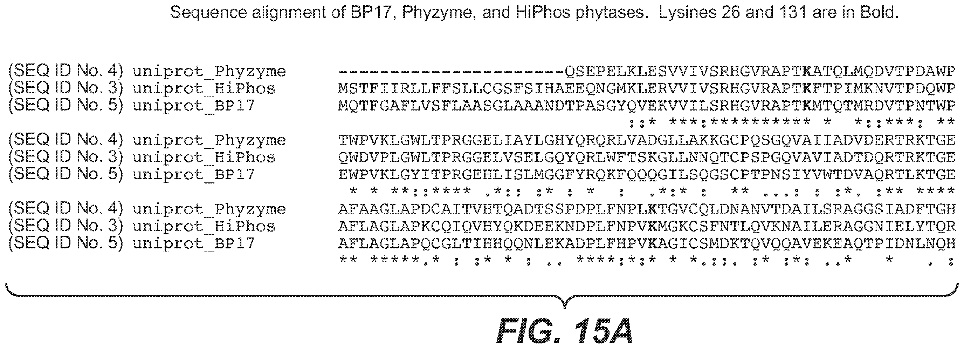

To explore the role of specific lysine amino acids contributing to glycation, and protein stability, protein engineering efforts were undertaken. This study was performed using WT and variants K131R and K26R. These lysine residues were chosen based on (1) conservation at these positions among other phytase sequences (Figure 15A), (2) proximity to the substrate binding site and (3) results from mass spectral analysis of phytase BP17 from previous studies.

Sequences for the proteins considered in Figure 15A are below.

>HiPhos

SEQ ID NO:3

MSTFIIRLLFFSLLCGSFSIHAEEQNGMKLERVVIVSRHGVRAPTKFTPIMKNVTPDQW
PQWDVPLGWLT

PRGGELVSELGQYQRLWFTSKGLLNNQTCPSPGQVAVIADTDQRTRKTGEAFLAGLA
PKCQIQVHYQKDEEKNDPLFNPVKMGKCSFNTLQVKNAILERAGGNIELYTQRYQSSF
RTLENVLNFSQSETCKTTEKSTKCTLPEALPSELKVTPDNVSLPGAWSLSSTLTEIFLL
QEAQGMPQVAWGRITGEKEWRDLLSLHNAQFDLLQRTPEVARSRATPLLDMIDTALL
TNGTTENRYGIKLPVSLLFIAGHDTNLANLSGALDLNWSLPGQPDNTPPGGELVFEKW
KRTSDNTDWVQVSFVYQTLRDMRDIQPLSLEKPAGKVDLKLIACEEKNSQGMCSLKS
FSRLIKEIRVPECAVTE

>Phyzyme

# SEQ ID NO:4

QSEPELKLESVVIVSRHGVRAPTKATQLMQDVTPDAWPTWPVKLGWLTPRGGELIAY
LGHYQRQRLVADGLLAKKGCPQSGQVAIIADVDERTRKTGEAFAAGLAPDCAITVHTQ
ADTSSPDPLFNPLKTGVCQLDNANVTDAILSRAGGSIADFTGHRQTAFRELERVLNFP
QSNLCLKREKQDESCSLTQALPSELKVSADNVSLTGAVSLASMLTEIFLLQQAQGMPE
PGWGRITDSHQWNTLLSLHNAQFYLLQRTPEVARSRATPLLDLIMAALTPHPPQKQAY
GVTLPTSVLFIAGHDTNLANLGGALELNWTLPGQPDNTPPGGELVFERWRRLSDNSQ
WIQVSLVFQTLQQMRDKTPLSLNTPPGEVKLTLAGCEERNAQGMCSLAGFTQIVNEA
RIPACSLRSHHHHHH

>BP17 ETD

SEQ ID NO:5

MQTFGAFLVSFLAASGLAAANDTPASGYQVEKVVILSRHGVRAPTKMTQTMRDVTPN
TWPEWPVKLGYITPRGEHLISLMGGFYRQKFQQQGILSQGSCPTPNSIYVWTDVAQR
TLKTGEAFLAGLAPQCGLTIHHQQNLEKADPLFHPVKAGICSMDKTQVQQAVEKEAQT
PIDNLNQHYIPSLALMNTTLNFSKSPWCQKHSADKSCDLGLSMPSKLSIKDNGNEVSL
DGAIGLSSTLAEIFLLEYAQGMPQAAWGNIHSEQEWALLLKLHNVYFDLMERTPYIARH
KGTPLLQAISNALNPNATESKLPDISPDNKILFIAGHDTNIANIAGMLNMRWTLPGQPDN
TPPGGALVFERLADKSGKQYVSVSMVYQTLEQLRSQTPLSLNQPAGSVQLKIPGCND
QTAEGYCPLSTFTRVVSQSVEPGCQLQ

The following materials were used: *Buttauxilla sp.* phytase variants were produced in *Trichoderma reseei* host and purified using ion exchange chromatography; BP17/BP111 Konelab Standard (22000 FTU/g; density=1.07); 402.4 mM glucose in water; Zeba 7 kDa Cutoff Desalting Plate; para-nitrophenylphosphate; and, Phytate / Malachite Green Reagents.

To induce the Maillard reaction, glucose stock solutions were prepared at several concentrations: 0, 8, 20 and 40 mM. Reactions were initiated by mixing 50 uL of phytase variant (2 mg/mL) with 50 uL of glucose solution in PCR strips. A PCR strip was prepared for each of four time points. Samples were frozen, lyophilized and placed in an incubator at 40C and 70% Relative Humidity (RH). Samples were removed at 0, 58, 100 and 237 hours and stored at -20°C until completion of the study. Afterwards, samples were resuspended with 100 uL of water and processed using a Zeba 7 kDa cutoff desalting plate to remove excess glucose.

Enzyme activities were determined using *p*NPP and IP6 substrates with BP17 enzyme standards. The *p*NPP assay relies on formation of the *p*-nitrophenylate ion, which absorbs at 405 nm. The IP6 assay is dependent on malachite green (MG) staining of the molybdate-phosphate complex, which absorbs at 620 nm.

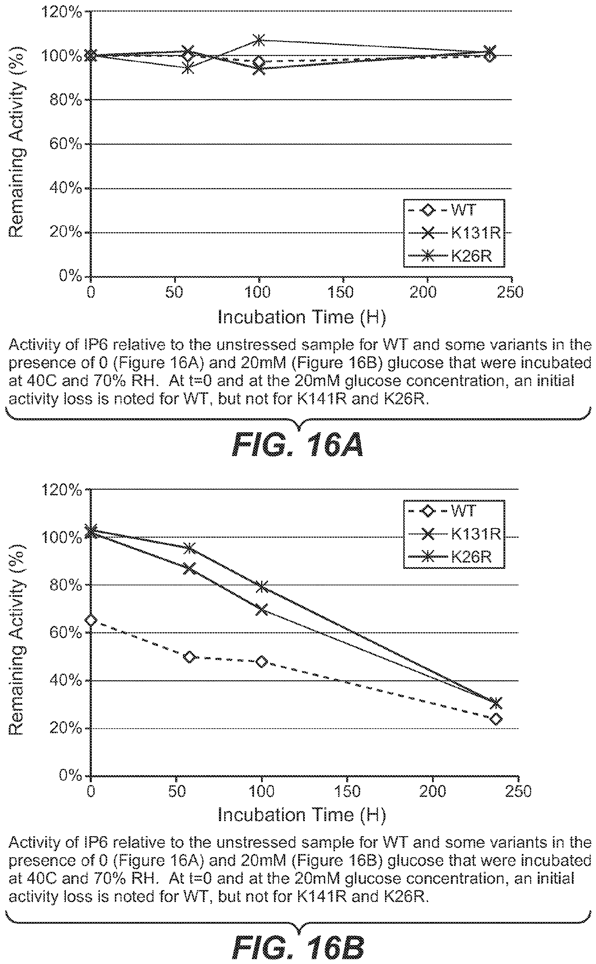

These data indicated that samples not treated with glucose retained full activity for both IP6 (Figure 16A) and *p*NPP (not shown) substrates throughout the duration of the stress test at 40C and 70%RH.

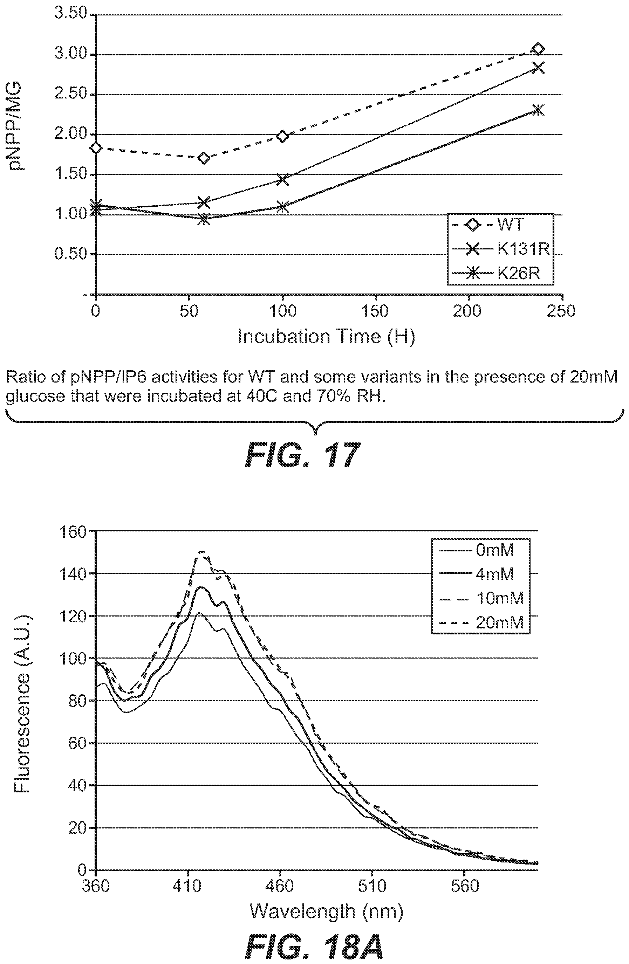

Further, retention of activity toward *p*NPP and loss of activity toward IP6 is an indication of the extent of lysine glycation due to the Maillard reaction. While the activity on *p*NPP is retained during glucose stress, activity on IP6 is diminished at initial time points (even at time zero) with an exception of K131 R, and to a lesser extent K26R (Figure 16B). The increase in the pNPP/MG ratio correlated with the concentration of glucose (Figure 17).

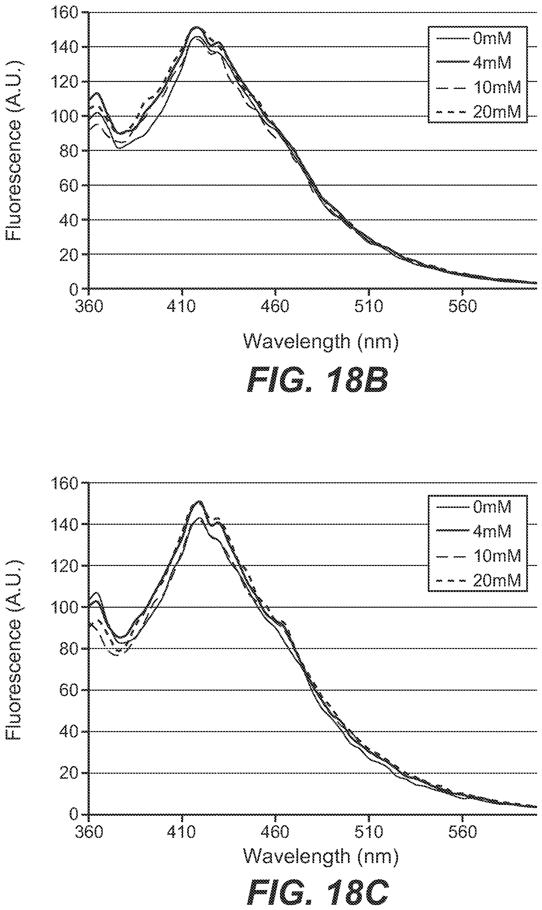

Also, at time 100 h, a glucose-dependent increase of intrinsic fluorescence is noted for WT, but not for K131R and K26R (Figures 18A-C). This increase of fluorescence at 415 nm is indicative of AGE formation in WT phytase. Intrinsic fluorescence spectra of WT phytase and its variants is shown in Figure 18A, K131R (Figure 18B), and K26R (Figure 18C), sampled after 100 h at 40°C and 70% RH. The glucose dependent increase in fluorescence signal at ~420 nm is a signature of the Maillard reaction (Journal of Dairy Science 94:5375-5380).

These results suggest that K131 and K26 are critical lysines that upon glucose stress alter IP6 substrate specificity. The fact that K131R and K26R variants are resistant to glucose stress while the wild type loses activity toward IP6 suggests that these residues undergo the Maillard reaction.

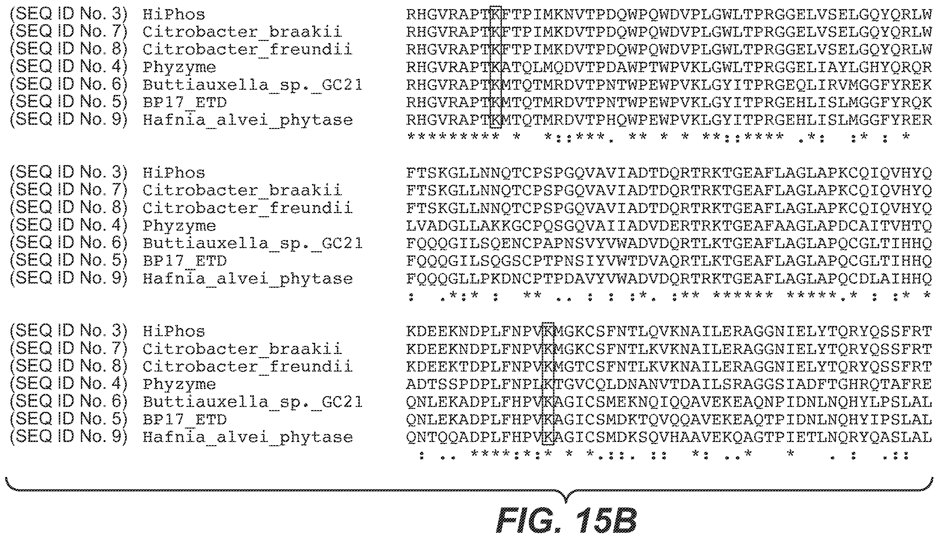

Additional sequences of interest that will be appreciated to benefit from the present teachings are shown in Figure 15B, and includes

SEQ ID NO: 6 gi|161898386|gb|ABX80238.1| phytase [Buttiauxella sp.
SEQ ID NO: 7 GC21];>gi|42795098|gb|AAS45884.1| phytase [Citrobacter braakii];
SEQ ID NO: 8 >gi|40748277|gb|AAR89622.1| phytase [Citrobacter freundii]; and,
SEQ ID NO: 9 >gi|364569314|gb|EHM46937.1| histidine acid phosphatase [Hafnia alvei ATCC 51873]

## Example 11

### Accelerated Stress Test:

Phytase granules (2 g), prepared according to the process described for Example 1 above, were weighed in individual specimen cups. Seven cups per sample were prepared, each cup representing a time point. Open cups were placed in incubators set to either 40°C and 70% Relative Humidity (RH), or 50°C and 70% RH. Cups containing samples were collected from the incubator at 0, 125, 305 and 335 h. Samples were allowed to equilibrate to ambient temperature and humidity for 20 minutes, covered with a lid and stored at -20°C until all samples were collected.

### Phytase Remaining Activity:

Granules (0.1 g) were extracted with -9.9 g assay buffer in 15 mL conical tubes. Samples were allowed to dissolve for 20 min at room temperature with 58 RPM rotation. Samples were centrifuged at 3000 RPM for 3 min, supernatant containing phytase was manually filtered. Phytase activity, for all extracted samples, was assumed to be ~120 FTU/mL and were diluted with MG assay buffer to 15 FTU/mL for pNPP assay and to 0.010 FTU/mL for IP6/Malachite Green assay.

### Glucose Level in Complete Granules:

Granules were grounded with mortar until a fine powder was obtained. Grinding process was performed in the hood to avoid enzyme exposure. Ground samples (2 g) were weighed in 15 mL conical tubes and 3 mL 2% TFA was added to the dry powder. Sample was mixed and allowed to dissolve for 15 min at room temperature by constant rotation at ~25 RPM. Sample (500 uL) was neutralized with 500 uL Tris (pH 7.6), mixed, centrifuged for 2 min at 15000 RPM and supernatant was analyzed for glucose using the Konelab glucose oxidase assay.

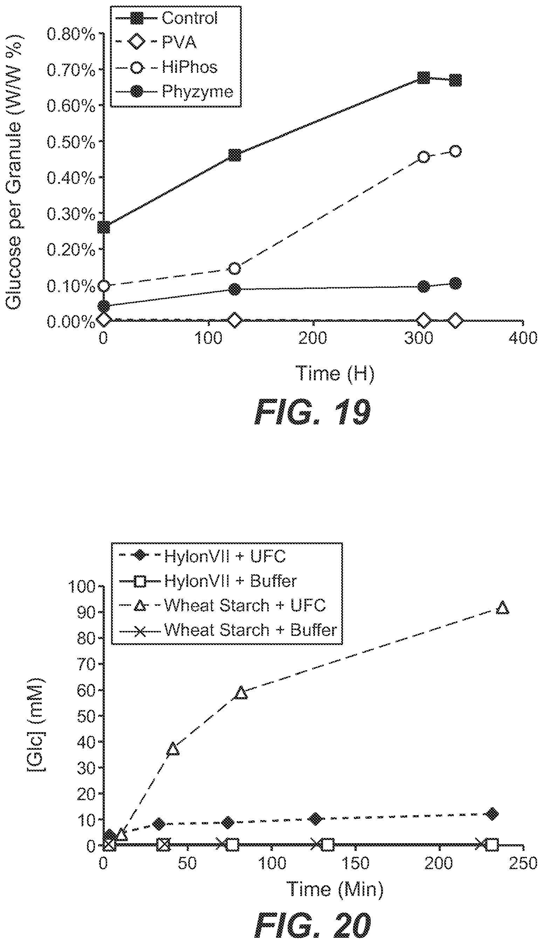

The results are depicted in Figure 19. Shown here is the concentration of glucose/granule in W/W% produced over time for Phyzyme, Hiphos, and different BP17-containing TPT granules made with diafiltered UFC in the presence of either starch/sucrose (control) or PVA in the enzyme matrix. Values are those for samples collected at 0, 125, 305 and 335 h post accelerated stressed test performed at 40C and 70% RH.

**Example 12-Resistant Starch as a TPT Formulation Component to Reduce Maillard Reaction**

Reducing sugars in solid formulations can be part of the formulation ingredient (e.g., dextrose) or produced inadvertently overtime from other components such as starch and sucrose due to background glucoamylase and invertase. While glucoamylase produces glucose from starch substrate, invertase produces glucose and fructose from the non-reducing sugar sucrose. Glucoamylase and invertase are produced ubiquitously along with the enzyme of interest in the production strain *Trichoderma reesei.* Thus, using starch and sucrose in solid formulations should be acceptable only if the enzyme of interest can sustain high levels of reducing sugars without losing enzyme activity.

To test the effect of a starch resistant to hydrolysis, glucose production was measured when Hylon VII was employed. Hylon VII is a resistant starch from a corn hybrid that contains 70% amylose and 30% amylopectin. It is predicted that glucose that would normally arise by the background glucoamylase present in diafiltered BP17 ETD UFC, would be reduced by the use of Hylon VII. Measurements were done at 15°C. Total glucose liberated from Hylon VII was compared to that liberated from wheat starch, a more conventional formulation ingredient that would be predicted to undergo hydrolysis by the glucoamylase.

**Materials:**

1. Axtra PHY/BP17 ETD UFC from Beloit (C14068; Batch 1681970737) (pH 4.9)
2. 250 mM Sodium Acetate (pH 4.9)
3. Hylon VII Starch
4. Wheat Starch from Hanko
5. Trifluoroacetic acid (TFA)
6. 500 mM Tris, pH 7.6
7. Glucose Oxidase Assay (Konelab)

**Glucose Production:**

Axtra PHY/BP17 ETD was diafiltered with three diavolumes. UFC and buffer were pre-chilled on ice prior to initiating reaction. Samples were transferred to a refrigerated incubator set to 15°C and mixed by an automatic rotator. Wheat starch and Hylon VII (3.7 g) were suspended independently in cold 16.3 g diafiltered UFC or 250 mM sodium acetate (pH 4.9). Starch concentration used is equivalent to that present in TPT formulation. Time points were taken for about 4 h and glucose production was halted by acid quench using TFA. The following time points were collected taking into account the quenching time:

Hylon VII UFC: 3.5, 32.7, 73.87, 126, 231.35 min

Hylon VII Buffer: 3.45, 35.18, 76.65, 133.35, 231.18 min

Wheat Starch UFC: 3.28, 33.9, 74.6, 129.51, 230.26 min

Wheat Starch Buffer: 2.93, 36.27, 70.4, 126.82, 224.67 min

The quench procedure was performed at room temperature as follows. Reaction mixture (1 mL) was spun at 15000 RPM for 2 min. Supernatant (0.49 mL) was collected and added to eppendorf tube containing 10 ul TFA. Solution was vortex to ensure proper mixing and centrifuged for 2 min at 15000 RPM to remove precipitate. Supernatant (400 ul) was added to 500 ul of 500 mM Tris, pH 7.6 in a new tube as a way to neutralize the solution prior glucose measurement. Samples were frozen at - 80C soon after the acid quench step and until glucose analysis.

To measure glucose, samples were thawed for 20-30 min at room temp and glucose concentration was determined following the Konelab glucose oxidase assay procedure. Although all UFC samples were in range of the assay, samples in buffer were below the limit of detection.

We observed that background glucoamylase present in BP17 ETD UFC produces 12 mM glucose from Hylon VII starch after 231 min at 15°C and pH 4.9. This concentration of glucose produced from Hylon VII is ~7.7-fold lower than that produced from wheat starch after 237 min.

The results are shown in Figure 20. Here a comparison is shown between glucose production from Hylon VII corn starch and wheat starch at 15C. The glucose produced is believed generally to arise by the action of background glucoamylase present in the BP17 ETD UFC. Glucose production was initiated by adding starch to either UFC or buffer (pH 4.9) that had been pre-equilibrated to 15°C. Time points were acid quenched followed by neutralization. Glucose produced in the quenched samples was measured using the glucose oxidase assay.

**Claims**

1. A method of improving enzyme stability comprising;

   making an enzyme in a fermentation, wherein the fermentation comprises a fermentation medium comprising problematic sugars glucose, fructose and/or sucrose and hydrolyzable starch, and wherein the enzyme comprises at least one glycatable amino acid residue and is an enzyme susceptible to problematic glycation;
   recovering the enzyme; and,
   removing sufficient amounts of the problematic sugars to improve enzyme stability relative to a similarly fermented enzyme lacking the removing,
   wherein the enzyme is selected from a phytase and a cellulase.

2. The method of claim 1 wherein the removing comprises diafiltering an ultrafiltrate concentrate to remove the problematic sugars to form an enzyme concentrate, optionally wherein the diafiltering comprises at least two, at least three, or at least four, diavolumes, such that the sugars content of the enzyme concentrate is reduced to less than 0.1% w/w.

3. The method of claim 1 wherein the removing comprises heating the enzyme after the fermentation at 50°C for at least 1, 2, 4, 8, or 12 hours to inactivate any starch hydrolysing enzymes present.

4. The method of claim 1 wherein the removing comprises a previous genetic manipulation, wherein at least one active site lysine residue was removed from the polypeptide, optionally wherein the active lysine residue is K131 and/or K26, and the enzyme is:

   (a) wild type *Buttiauxella sp.* Phytase, or
   (b) BP17 (SEQ ID NO: 1).

5. An engineered phytase wherein an amino acid residue at position 131 and/or 26 relative to wild-type *Buttiauxella* phytase is arginine.

**Patentansprüche**

1. Verfahren zum Verbessern von Enzymstabilität, Folgendes umfassend :

   Herstellen eines Enzyms in einer Fermentation, wobei die Fermentation ein Fermentationsmedium umfasst, das die problematischen Zucker wie Glucose, Fructose und/oder Saccharose, sowie hydrolisierbare Stärke umfasst, und wobei das Enzym mindestens einen glykierbaren Aminosäurerest umfasst und ein Enzym darstellt, welches anfällig für problematische Glykation ist;
   Rückgewinnung des Enzyms; und
   Entfernen von ausreichenden Mengen der problematischen Zucker, um die Enzymstabilität zu verbessern, im Vergleich zu einem ähnlich fermentierten Enzym, ohne diese Entfernung;
   wobei das Enzym ausgewählt ist aus einer Phytase und einer Cellulase.

2. Verfahren nach Anspruch 1, wobei das Entfernen das Diafiltrieren eines Ultrafiltratkonzentrats umfasst, um die problematischen Zucker zu entfernen und ein Enzymkonzentrat zu bilden, wahlweise wobei das Diafiltrieren mindestens zwei, mindestens drei oder mindestens vier Diavolumen umfasst, so dass der Zuckergehalt des Enzymkonzentrats auf weniger als 0,1% Gew./Gew. reduziert wird.

3. Verfahren nach Anspruch 1, wobei das Entfernen das Erhitzen des Enzyms nach der Fermentierung auf 50°C für mindestens 1, 2, 4, 8 oder 12 Stunden umfasst, um jegliche vorhandenen Stärke hydrolisierenden Enzyme zu inaktivieren.

4. Verfahren nach Anspruch 1, wobei das Entfernen vorausgegangene genetische Manipulation umfasst, wobei mindestens ein Aktivzentrum-Lysinrest von dem Polypeptid entfernt wurde, wahlweise wobei der aktive Lysinrest K131 und/oder K26 ist und das Enzym Folgendes ist:

(a) Wildtyp-*Buttiauxella*-sp.-Phytase, oder
(b) BP17 (SEQ ID NO:1).

5. Modifizierte Phytase, wobei ein Aminosäurerest an Position 131 und/oder 26 bezüglich der Wildtyp-*Buttiauxella*-Phytase Arginin ist.

**Revendications**

1. Procédé d'amélioration de la stabilité enzymatique comprenant:

préparer une enzyme dans une fermentation, la fermentation comprenant un milieu de fermentation comprenant des sucres problématiques comme le glucose, le fructose et / ou le saccharose et de l'amidon hydrolysable, et l'enzyme comprenant au moins un résidu d'acide aminé pouvant être glyqué et étant une enzyme sensible à la glycation problématique;
récupérer l'enzyme; et
l'élimination de quantités suffisantes des sucres problématiques pour améliorer la stabilité de l'enzyme par rapport à une enzyme fermentée de la manière similaire, mais sans élimination,
dans lequel l'enzyme est choisie parmi une phytase et une cellulase.

2. Procédé selon la revendication 1, dans lequel l'élimination comprend la diafiltration d'un concentré d'ultrafiltrat pour éliminer les sucres problématiques afin de former un concentré d'enzyme, le diafiltrage comprenant optionnellement au moins deux, au moins trois ou au moins quatre diavolumes, tels que la teneur en sucres du concentré d'enzyme est réduite à moins de 0,1% p/p.

3. Procédé selon la revendication 1, dans lequel l'élimination comprend le chauffage de l'enzyme après la fermentation à 50 ° C pendant au moins 1, 2, 4, 8 ou 12 heures pour inactiver toute enzyme d'hydrolyse de l'amidon présente.

4. Procédé selon la revendication 1, dans lequel l'élimination comprend une manipulation génétique antérieure, dans laquelle au moins un résidu de lysine de site actif a été enlevé du polypeptide, éventuellement dans lequel le résidu de lysine actif est K131 et/ou K26, et l'enzyme est:

(a) phytase de *Buttiauxella sp.* de type sauvage, ou
(b) BP17 (SEQ ID N°: 1).

5. Phytase de synthèse dans laquelle un résidu d'acide aminé en position 131 et/ou 26 par rapport à la phytase de *Buttiauxella* de type sauvage est l'arginine.

# Product Activity Loss – Mo-V Assay

### BP 17 ETD at 25°C

### BP 17 ETD at 40°C

## FIG. 1A

## Product Activity Loss – pNPP Assay

### Phytase B, 25°C

### Phytase B, 40°C

## *FIG. 1B*

**BP17 ETD Spray 1 - 5 Days**

"Spray 1" Lot #1661821166
Control - 25°C and 40% RH

"Spray 1" Lot #20104026
Control - 25°C and 40% RH

"Spray 1" Lot #1661821166
50°C and 70% RH

"Spray 1" Lot #20104026
50°C and 70% RH

*FIG. 2A*

**BP17 ETD Cedar UFC with Add Glucose after 12 Days**

UFC Lot #1661821166
Control - 25°C and 40% RH

UFC Lot #1661821166
+ 1 mg/mL Glucose
Control - 25°C and 40% RH

UFC Lot #1661821166
+ 10 mg/mL Glucose
Control - 25°C and 40% RH

UFC Lot #1661821166
50°C and 70% RH

UFC Lot #1661821166
+ 1 mg/mL Glucose
50°C and 70% RH

UFC Lot #1661821166
+ 10 mg/mL Glucose
50°C and 70% RH

*FIG. 2B*

EP 3 068 879 B1

Regular vs. Diafiltered BP17 ETD UFC - 7 Days Storage

Lot #20104026
Control - 25°C and 40% RH

Diafiltered Lot #20104026
Control - 25°C and 40% RH

Lot #20104026
50°C and 70% RH

Diafiltered Lot #20104026
50°C and 70% RH

FIG. 2C

**Regular vs. Diafiltered BP17 ETD UFC - 7 Days Storage**
Diafiltration of UFC using a 10K membrane removes all sugars and browning reaction is arrested.

*FIG. 2D*

EP 3 068 879 B1

## Intrinsic Fluorescence Directly Correlates with Activity Loss

### Fluorescence Emission Spectra of BP17 ETD Extracted from Granules

**FIG. 3**

## Fluorescamine Labeling of Lysines Tracks with Activity Loss

### Fluorescamine Reaction with Free Amines

**FIG. 4**

## Inverse Correlation Between Sample Fluorescence and Free Lysines

Summary of intrinsic fluorescence and fluorescamine fluorescence
as a function of storage temperature

*FIG. 5*

*FIG. 6*

## FIG. 7

<br>

FIG. 7A

FIG. 7B

# FIG. 7A

MS Evidence for Glycation

| Digest | Lysine | TPT t=0 | Peptide | % Glycation |
|---|---|---|---|---|
| Asn-N/ Chymo | K[12] | 9 – 16 | Y.QVEKVVIL.S + Hex (K) | 29 |
| | K[97] | 97 – 102 | L.KTGEAF.L + Hex (K) | 54 |
| | K[122] | 113 – 123 | L.TIHHQQNLEKA.D + Hex (K) | 23 |
| | K[148] | 144 – 154 | Q.QAVEKEAQTPI.D + Hex (K) | 26 |
| | K[290] | 281 – 292 | N.ALNPNATESKLP.D + HexNAc (N); Hex (K) | 24 |
| | K[380] | 380 – 385 | L.KIPGCN.D + Hex (K) | 19 |

| Digest | Lysine | TPT t=6 mo. @ 40C | Peptide | % Glycation |
|---|---|---|---|---|
| Asn-N/ Chymo | K[12] | 9 – 16 | Y.QVEKVVIL.S + Hex (K) | 69 |
| | K[97] | 97 – 102 | L.KTGEAF.L + Hex (K) | 72 |
| | K[122] | 113 – 123 | L.TIHHQQNLEKA.D + Hex (K) | 67 |
| | K[139or148] | 138 – 154 | M.DKTQVQQAVEKEAQTPI.D + 1 Hex (K) | 78 |
| | K[139and148] | 138 – 154 | M.DKTQVQQAVEKEAQTPI.D + 2 Hex (K) | 81 |
| | K[202] | 200 – 210 | L.SIKDNGNEVSL.D + Hex (K); Deamidated (NQ) | 60 |
| | K[290] | 281 – 292 | N.ALNPNATESKLP.D + HexNAc (N); Hex (K) | 68 |
| | K[380] | 380 – 385 | L.KIPGCN.D + Hex (K) | 68 |

<br>

| Digest | Lysine | TPT t=0 | Peptide | % Glycation |
|---|---|---|---|---|
| Trypsin | K[131] | 124 – 137 | A.DPLFHPVKAGICSM.D + Hex (K) | N/A |
| | K[139or148] | 138 – 148 | M.DKTQVQQAVEK.E + Hex (K) | N/A |
| | K[299] | 297 – 306 | P.DNKILFIAGH.D + Hex (K) | N/A |

| Digest | Lysine | TPT t=6 mo. @ 40C | Peptide | Fold increase relative to t=0 |
|---|---|---|---|---|
| Trypsin | K[12] | 2 – 18 | N.DTPASGYQVEKVVILSR.H + Hex (K) | >110 |
| | K[131] | 124 – 137 | A.DPLFHPVKAGICSM.D + Hex (K) | 30 |
| | K[139or148] | 138 – 148 | M.DKTQVQQAVEK.E + Hex (K) | 1 |
| | K(198or202] | 190 – 202 | C.DLGLSMPSKLSIK.D + 1 Hex (K) | 20 |
| | K(198and202] | 190 – 202 | C.DLGLSMPSKLSIK.D + 2 Hex (K) | >100 |
| | K[270] | 269 – 290 | R.HKGTPLLQAISNALNPNATESK.L + Hex (K) | >80 |
| | K[299] | 297 – 306 | P.DNKILFIAGH.D + Hex (K) | 5 |
| | K[380] | 364 – 385 | R.SQTPLSLNQPAGSVQLKIPGCN.D + Hex (K) | >110 |

*FIG. 7B*

EP 3 068 879 B1

Some Lysines Near Binding Site

FIG. 8

Induced Glycation in Lyophilized Samples: Pure BP17 ETD

• Reaction Conditions:
    2 mg/mL Pure Phytase
    0-1 M Sugar (Glucose, Sucrose and Fructose)

*FIG. 9*

FIG. 10

**Effect of Diafiltration and Heat Treatment on Accelerated Storage Stability of Lyophilized Phytase**

*FIG. 11*

**Accelerated Storage Stability at 50°C, 70% RH**

FIG. 12

Effect Glucose Concentration on Storage Stability of
Lyophilized Cellulase Enzyme at 50°C and 70% RH

FIG. 13

Intrinsic Fluorescence of Cellulase after Storage at 50°C and 70% RH

FIG. 14

Sequence alignment of BP17, Phyzyme, and HiPhos phytases.  Lysines 26 and 131 are in Bold.

```
(SEQ ID No. 4) uniprot_Phyzyme    -------------------------------QSEPELKLESVVIVSRHGVRAPTKATQLMQDVTPDAWP
(SEQ ID No. 3) uniprot_HiPhos     MSTFIIRLLFFSLLCGSFSIHAEEQNGMKLERVVIVSRHGVRAPTKFTPIMKNVTPDQWP
(SEQ ID No. 5) uniprot_BP17       MQTFGAFLVSFLAASGLAAANDTPASGYQVEKVVILSRHGVRAPTKMTQTMRDVTPNTWP
                                           :.:*  ***:********** *   *:.***: **

(SEQ ID No. 4) uniprot_Phyzyme    TWPVKLGWLTPRGGELIAYLGHYQRQRLVADGLLAKKGCPQSGQVAIIADVDERTRKTGE
(SEQ ID No. 3) uniprot_HiPhos     QWDVPLGWLTPRGGELVSELGQYQRLWFTSKGLLNNQTCPSPGQVAVIADTDQRTRKTGE
(SEQ ID No. 5) uniprot_BP17       EWPVKLGYITPRGEHLISLMGGFYRQKFQQQGILSQGSCPTPNSIYVWTDVAQRTLKTGE
                                   * * **:.:****  .*:: .:* : *  :  .*:* :  ** ...: : :*. :** ****

(SEQ ID No. 4) uniprot_Phyzyme    AFAAGLAPDCAITVHTQADTSSPDPLFNPLKTGVCQLDNANVTDAILSRAGGSIADFTGH
(SEQ ID No. 3) uniprot_HiPhos     AFLAGLAPKCQIQVHYQKDEEKNDPLFNPVKMGKCSFNTLQVKNAILERAGGNIELYTQR
(SEQ ID No. 5) uniprot_BP17       AFLAGLAPQCGLTIHHQQNLEKADPLFHPVKAGICSMDKTQVQQAVEKEAQTPIDNLNQH
                                   ** *****.*  :  :* *  :  ..  ****:*:* *  *.::.  :* :*: ..*    *    . :
```

FIG. 15A

```
(SEQ ID No. 3)  HiPhos               RHGVRAPTKFTPIMKNVTPDQWPQWDVPLGWLTPRGGELVSELGQYQRLW
(SEQ ID No. 7)  Citrobacter_braakii  RHGVRAPTKFTPIMKDVTPDQWPQWDVPLGWLTPRGGELVSELGQYQRLW
(SEQ ID No. 8)  Citrobacter_freundii RHGVRAPTKFTPIMKDVTPDQWPQWDVPLGWLTPRGGELVSELGQYQRLW
(SEQ ID No. 4)  Phyzyme              RHGVRAPTKATQLMQDVTPDAWPTWPVKLGWLTPRGGELIAYLGHYQRQR
(SEQ ID No. 6)  Buttiauxella_sp._GC21 RHGVRAPTKMTQTMRDVTPNTWPEWPVKLGYITPRGEQLIRVMGGFYREK
(SEQ ID No. 5)  BP17_ETD             RHGVRAPTKMTQTMRDVTPNTWPEWPVKLGYITPRGEHLISLMGGFYRQK
(SEQ ID No. 9)  Hafnia_alvei_phytase RHGVRAPTKMTQTMRDVTPHQWPEWPVKLGYITPRGEHLISLMGGFYRER
                                     ********* *  *:;***. ** * * **;;**** .*:  :* : *


(SEQ ID No. 3)  HiPhos               FTSKGLLNNQTCPSPGQVAVIADTDQRTRKTGEAFLAGLAPKCQIQVHYQ
(SEQ ID No. 7)  Citrobacter_braakii  FTSKGLLNNQTCPSPGQVAVIADTDQRTRKTGEAFLAGLAPKCQIQVHYQ
(SEQ ID No. 8)  Citrobacter_freundii FTSKGLLNNQTCPSPGQVAVIADTDQRTRKTGEAFLAGLAPKCQIQVHYQ
(SEQ ID No. 4)  Phyzyme              LVADGLLAKKGCPQSGQVAIIADVDERTRKTGEAFAAGLAPDCAITVHTQ
(SEQ ID No. 6)  Buttiauxella_sp._GC21 FQQQGILSQENCPAPNSVYVWADVDQRTLKTGEAFLAGLAPQCGLTIHHQ
(SEQ ID No. 5)  BP17_ETD             FQQQGILSQGSCPTPNSIYVWTDVAQRTLKTGEAFLAGLAPQCGLTIHHQ
(SEQ ID No. 9)  Hafnia_alvei_phytase FQQQGLLPKDNCPTPDAVYVWADVDQRTRKTGEAFLAGLAPQCDLAIHHQ
                                     :  .*:* :  ** .. : : :*. :** ***** *****.* : :* *


(SEQ ID No. 3)  HiPhos               KDEEKNDPLFNPVKMGKCSFNTLQVKNAILERAGGNIELYTQRYQSSFRT
(SEQ ID No. 7)  Citrobacter_braakii  KDEEKNDPLFNPVKMGKCSFNTLKVKNAILERAGGNIELYTQRYQSSFRT
(SEQ ID No. 8)  Citrobacter_freundii KDEEKTDPLFNPVKMGTCSFNTLKVKNAILERAGGNIELYTQRYQSSFRT
(SEQ ID No. 4)  Phyzyme              ADTSSPDPLFNPLKTGVCQLDNANVTDAILSRAGGSIADFTGHRQTAFRE
(SEQ ID No. 6)  Buttiauxella_sp._GC21 QNLEKADPLFHPVKAGICSMEKNQIQQAVEKEAQNPIDNLNQHYLPSLAL
(SEQ ID No. 5)  BP17_ETD             QNLEKADPLFHPVKAGICSMDKTQVQQAVEKEAQTPIDNLNQHYIPSLAL
(SEQ ID No. 9)  Hafnia_alvei_phytase QNTQQADPLFHPVKAGICSMDKSQVHAAVEKQAGTPIETLNQRYQASLAL
                                     :  .. ****:*:* * *.::. :: *: ..*  *   . : .::
```

*FIG. 15B*

Activity of IP6 relative to the unstressed sample for WT and some variants in the presence of 0 (Figure 16A) and 20mM (Figure 16B) glucose that were incubated at 40C and 70% RH. At t=0 and at the 20mM glucose concentration, an initial activity loss is noted for WT, but not for K141R and K26R.

## FIG. 16A

Activity of IP6 relative to the unstressed sample for WT and some variants in the presence of 0 (Figure 16A) and 20mM (Figure 16B) glucose that were incubated at 40C and 70% RH. At t=0 and at the 20mM glucose concentration, an initial activity loss is noted for WT, but not for K141R and K26R.

## FIG. 16B

Ratio of pNPP/IP6 activities for WT and some variants in the presence of 20mM glucose that were incubated at 40C and 70% RH.

## FIG. 17

## FIG. 18A

*FIG. 18B*

*FIG. 18C*

**FIG. 19**

**FIG. 20**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9854980 A
- WO 9739116 A
- WO 2007044968 A
- EP 1996028 A
- WO 2011117397 A
- US 2010083392 A1
- WO 2009129489 A2
- GB 2167758 A
- WO 2013119468 A
- WO 2007113292 A2
- EP 0231729 A1
- US 4371557 A
- US 4689297 A
- US 5324649 A
- EP 656058 B1
- US 454332 A
- US 6248706 B
- EP 804532 B1
- US 6534466 B
- US 61595923
- US 61595941 B
- US 20060193897 A1
- US 7018821 B
- US 8076113 B
- US 4106991 A
- US 4740469 A
- US 20130244415 W
- WO 2004035070 A1
- US 7713725 B2

**Non-patent literature cited in the description**

- **KLIBANOV.** Stabilization of Enzymes against Thermal Inactivation. *Advances in Applied Microbiology,* 1983, vol. 29, 1-28
- **RONDEAU et al.** The glycation of albumin: Structural and functional impacts. *Biochimie,* 2010
- **SAMBROOK et al.** Molecular Cloning: A Laboratory Manual. 1989
- Oligonucleotide Synthesis. 1984
- Current Protocols in Molecular Biology. 1994
- PCR: The Polymerase Chain Reaction. 1994
- Gene Transfer and Expression: A Laboratory Manual. Kriegler, 1990
- Pelleting Cost Center. **FAIRFIELD, D.** Feed Manufacturing Technology IV. American Feed Industry Association, 1994, 110-139
- **SINGLETON et al.** Dictionary of Microbiology and Molecular Biology. John Wiley and Sons, 1994
- **HALE ; MARKHAM.** The Harper Collins Dictionary of Biology. Harper Perennial, 1991
- **THOMPSON et al.** *Nucleic Acids Res.,* 1994, vol. 22, 4673-4680
- *Journal of Dairy Science,* vol. 94, 5375-5380